(19) Europäisches Patentamt
European Patent Office
Office européen des brevets

(11) **EP 2 148 337 B1**

(12) **EUROPÄISCHE PATENTSCHRIFT**

(45) Veröffentlichungstag und Bekanntmachung des Hinweises auf die Patenterteilung:
**24.06.2015 Patentblatt 2015/26**

(51) Int Cl.:
*H05B 3/34* *(2006.01)*   *H05B 3/84* *(2006.01)*

(21) Anmeldenummer: **09165615.7**

(22) Anmeldetag: **16.07.2009**

(54) **Flexibles beheiztes Flächenelement**

Flexible heated area element

Elément de surface chauffé et flexible

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **24.07.2008 DE 102008034748**

(43) Veröffentlichungstag der Anmeldung:
**27.01.2010 Patentblatt 2010/04**

(73) Patentinhaber: **tesa SE**
**20253 Hamburg (DE)**

(72) Erfinder:
• **Dietz, Bernd**
**22949, Ammersbek (DE)**

• **Keite-Telgenbüscher, Dr. Klaus**
**22529, Hamburg (DE)**
• **Ellringmann, Dr. Ute**
**22589 Hamburg (DE)**
• **Junghans, Monika**
**22457 Hamburg (DE)**
• **Domann, Frank**
**25436, Uetersen (DE)**
• **Dominikat, Udo**
**24999, Wees (DE)**

(56) Entgegenhaltungen:
**WO-A1-2008/098847   DE-A1- 10 310 722**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Beschreibung

Die Erfindung, welche durch die Merkmale des unabhängigen Anspruchs 1 definiert ist, betrifft ein Flächenelement mit einer ersten selbstklebenden Seitenfläche und einer zweiten selbstklebenden Seitenfläche, wobei das Flächenelement eine Schichtabfolge aus einer Heizschicht, einer Kontaktierungsschicht und einer Klebemassenschicht aufweist, in der die Heizschicht mit einer ersten Seitenfläche der Kontaktierungsschicht in Kontakt steht und mit dieser elektrisch leitend verbunden ist, und in der die Klebemasseschicht mit einer zweiten Seitenfläche der Kontaktierungsschicht in Kontakt steht, und wobei die Heizschicht aus einer intrinsisch erwärmbaren ersten Selbstklebemasse besteht, die als bei Hindurchleiten eines elektrischen Stroms sich erwärmender Kaltleiter ausgebildet ist, und in der die Klebemasseschicht aus einer zweiten Selbstklebemasse besteht. Die Erfindung betrifft ferner einen Verklebungsverbund aus einem derartigen Flächenelement und einem Verklebungssubstrat, ein Verfahren zur Herstellung des Flächenelements sowie die Verwendung dieses Flächenelements zur Verklebung von Verklebungssubstraten in der Automobilindustrie sowie zum Beheizen eines Verklebungsverbunds.

In vielen Bereichen werden zum Beheizen von Gegenständen oder Räumen Elektroheizungen eingesetzt. In Elektroheizungen wird Wärme als thermische Energie durch Umwandlung von elektrischer Energie (einschließlich magnetischer Energie) gewonnen. Elektroheizungen können grundsätzlich auf unterschiedlichen technischen Prinzipen beruhen.

Neben der Wärmeerzeugung auf der Grundlage von kapazitiven oder induktiven Effekten oder von elektromagnetischen Strahlen haben sich Heizungssysteme durchgesetzt, die ein Heizwiderstandselement enthalten (so genannte Widerstandsheizungen). In derartigen Systemen wird die bei Hindurchleiten eines elektrischen Stroms durch das Heizwiderstandselement entstehende Wärmeenergie (Joulesche Wärme) genutzt. Grundsätzlich kann dabei jeder elektrische Leiter als Heizwiderstandselement verwendet werden, der einen von null verschiedenen endlichen Widerstandswert aufweist.

Die Auswahl des Heizwiderstandselements ergibt sich aus der zu erzielenden Wärmeleistung; diese ist von dem Widerstandswert des Heizwiderstandselements und von dem durch das Heizwiderstandselement fließenden elektrischen Strom abhängig und nach dem Ohmschen Gesetz somit von der anliegenden Spannung. Demzufolge erfolgt die Auswahl des Heizwiderstandselements entsprechend der Beschaffenheit der darin vorhandenen Leitungswege, insbesondere entsprechend deren Querschnitt, Länge, spezifischen Widerstand und thermischer Belastbarkeit.

Insbesondere in der Automobilindustrie kommen vermehrt Widerstandsheizungen zum Einsatz, etwa zum Beheizen von Autositzen, von Fensterscheiben und von Automobilaußenspiegeln. Um bei derartigen Anwendungen die gewünschte Beheizung zu realisieren, werden im einfachsten Fall Widerstandsdrähte planar verlegt. Die Heizleistung ist dabei konstant und wird über einen externen Mechanismus geregelt.

In den letzten Jahren haben sich hierbei so genannte PTC-Elemente als Heizwiderstandselemente durchgesetzt. Ein PTC-Element ist ein Heizwiderstandselement, dessen den Strom leitende Bereiche aus einem Material bestehen, die einen positiven Temperaturkoeffizienten (englisch: positive temperature coefficient, PTC) aufweisen; diese Materialien werden auch als Kaltleiter bezeichnet.

Kaltleiter sind daher den elektrischen Strom leitende Materialien, deren Widerstand mit der Temperatur ansteigt und die somit bei tieferen Temperaturen den Strom besser leiten als bei hohen Temperaturen. Die Verwendung derartiger sich kaltleitend verhaltender Materialien als Heizwiderstandselemente (PTC-Elemente) bietet den Vorteil, dass bei Anlegen einer konstanten Spannung an ein solches Heizelement ein Überhitzen des Heizelements vermieden wird, da bei einem Anstieg der Betriebstemperatur der Widerstand des Heizelements zunimmt, wodurch gemäß dem Ohmschen Gesetz der Strom sich proportional zu der Widerstandszunahme verringert, die insgesamt erzeugte Wärmeleistung abnimmt und das Heizelement sich wieder abkühlt. Je nach dem konkreten Anwendungszweck kann eine derartige intrinsische Regelung zur Temperaturbegrenzung anstelle von oder zusätzlich zu einer externen Regelung eingesetzt werden.

Auch im Automobilbau hat sich die Verwendung von derartigen PTC-Elementen durchgesetzt. So werden für Automobilaußenspiegel beispielsweise mit Aluminium-Leiterflächen kontaktierte PTC-Elemente verklebt, die die Rückseite des Spiegelglases mit einer Trägerplatte im Halter des Automobilaußenspiegels verbinden. Wird nun eine Spannung an das PTC-Element angelegt, so heizt sich dieses infolge des Stromflusses auf. Die dabei erzeugte Wärme wird über ein doppelseitiges Haftklebeband auf die Glasoberfläche des Spiegels übertragen und beheizt diesen somit. Auf diese Weise lassen sich auf der Glasoberfläche des Spiegels Temperaturen von 45 °C bis 80 °C erzielen.

Als Kaltleiter werden in einem solchen verklebbaren Heizelement in der Regel rußhaltige teilkristalline Thermoplaste wie etwa Polyethylen, Polyvinylidenfluorid, Hexafluorpropylen oder Tetrafluorethylen verwendet. Der Stand der Technik ist in DE 29 48 350 A1, EP 0 307 205 A1, EP 0 512 703 A1 sowie EP 0 852 801 A1 detailliert beschrieben. In der Verwendung als Spiegelheizung werden diese Kaltleiter in Form einer Tinte auf eine durchgängige Leiterfläche aufgebracht, die als elektrische Kontaktierungselektrode dient und die auf einer separaten Trägerfolie einer Stärke von üblicherweise 75 $\mu$m bis 250 $\mu$m angeordnet sind. Das in der Tinte enthaltene Lösungsmittel wird in einem abschließenden Trocknungsschritt entfernt. Derartige Tinten sind in EP 0 435 923 A1 ausführlich beschrieben.

Das bestehende System aus Trägerfolie mit Leiterflächen und intrinsisch heizbarem Lack ist hinreichend, um eine Erwärmung zu erzielen, erfordert aber einen relativ komplizierten Aufbau, da die einzelnen Komponenten des Heizelements nicht nur mit dem Glas des Spiegels verklebt werden müssen sondern auch mit der Trägerplatte des Spiegels, die in vielen Fällen aus dem Kunststoff Acrylnitril/Butadien/Styrol (ABS) besteht. Die Verklebung dieser unterschiedlichen Materialien stellt dabei besondere Anforderungen an das Klebeband.

Neben den Aspekten, die in den Materialien des jeweiligen Haftgrundes begründet liegen, müssen bei einem solchen Haftklebeband, das zur Befestigung des Heizelements an der Spiegelplatte verwendet wird und das die Wärme vom Heizelement zur Spiegeloberfläche transportiert, zusätzlich zu einer möglichst hohen thermischen Leitfähigkeit auch besondere Anpassungen im Hinblick auf die Wärmescherfestigkeit bei erhöhten Temperaturen sowie die Witterungsbeständigkeit und Haftklebrigkeit bei tiefen Temperaturen erfüllt sein. Dies gilt ebenfalls für eine separate Klebeschicht, die zum Befestigen des Verbundsbands aus Trägerfolie und leitfähiger Tinte an der Trägerplatte der Spiegelhalterung vorgesehen ist.

Weitere Anforderungen an das Verbundband aus Klebeband, Trägerfolie mit Leiterflächen und intrinsisch heizbarem Lack betreffen die Funktionalität dieses Verbundbands als Splitterschutz (so genanntes Anti-Splintering), wobei das Verbundband ein Freisetzen von Splittern bei einem eventuellen Bruch des Spiegels wirksam verhindern soll. Dies wird in der Regel mit dem Einsatz einer weiteren Trägerfolie im Klebeband erreicht.

Ein solches Verbundband weist insgesamt jedoch eine allenfalls geringe Flexibilität auf. Daher besitzen die aus dem Stand der Technik bekannten, als Verbundband verklebbaren Heizelemente den Nachteil, dass diese relativ starr sind. Das Verbundband haftet somit auf einem gekrümmten Haftgrund nur schlecht, da die Festigkeit des Verbundbands einer Verformung einen hohen mechanischen Widerstand entgegenbringt. Hierdurch kann es zu einem lokalen oder vollflächigen Ablösen des Heizelements vom Verklebungssubstrat (dem Haftgrund oder Träger) kommen, was die Übertragung der elektrisch erzeugten Wärmeenergie auf das Verklebungssubstrat vermindert oder sogar verhindert. Darüber hinaus beeinträchtigt die Starrheit des Aufbaus herkömmlicher verklebbarer Heizelemente die mechanische Kälteschlagfestigkeit einer Verklebung unterschiedlicher Verklebungssubstrate, etwa eines Spiegels mit einem Spiegelhalter.

Insbesondere bei großen und gewölbten Substratflächen ergibt sich das Problem, dass durch Fertigungstoleranzen (etwa von Spiegelglas und Trägerplatte) differierende Spaltmaße über die Fläche auftreten, die oft eine vollflächige Klebeverbindung verhindern. In diese Bereiche können zudem flüssige oder gasförmige Medien (Fluide) eindringen, beispielsweise Regenwasser oder Kondenswasser, und die Festigkeit der Klebeverbindung weiter herabsetzen.

Dieser Effekt ist insbesondere bei Autorückspiegeln problematisch, die einen einteiligen Spiegel mit einem erweiterten Sichtbereich aufweisen, in denen der Spiegel eine in zwei Raumrichtungen gekrümmte Oberfläche aufweist (Weitwinkelspiegel oder Nahbereichsspiegel). Eine Verklebung des Spiegels mit dem verklebbaren Heizelement wird bei einer derartigen Krümmung in zwei Dimensionen durch die starre Trägerfolie verhindert, auf die die Leiterflächenstrukturen aufgebracht sind. Zusätzlich zu der Trägerfolie erschweren auch die Leiterflächen die Verklebung auf einem gekrümmten Substrat, da diese aus vergleichsweise starren Metallschichten, leitfähigen Lacken oder Druckfarben bestehen, die bei starkem Biegen oder Dehnen brechen können, so dass der elektrische Kontakt in diesen Systemen nicht zuverlässig gewährleistet ist.

Darüber hinaus ergibt sich bei modernen Automobilaußenspiegelaufbauten das Problem, dass zusätzlich zu dem verklebbaren Heizelement weitere Funktionalitäten im Automobilaußenspiegel realisiert werden sollen (etwa eine elektrochrome Abtönung des Spiegels), deren Realisierung ebenfalls zur Einbautiefe oder Gesamtstärke des Bauteils beiträgt. Infolge solcher durch die geforderten funktionalen Strukturen immer dickeren Funktions- und Verklebungsaufbauten zwischen dem eigentlichen Spiegelglas und der Trägerplatte wird der Spielraum des Designers bei der Gestaltung des Autospiegels deutlich eingeschränkt und zudem das Gewicht des Automobilaußenspiegels insgesamt vergrößert.

Eine Verbesserung ließ sich dadurch erzielen, dass die elektrisch leitenden Strukturen des Heizelements mit dem Klebeband in einem Flächenelement gemeinsam realisiert sind. Ein derartiges haftklebriges Flächenelement, das intrinsisch erwärmbar ist und die Heizfunktion mit der Haftklebrigkeit verbindet, ist in DE 103 10 722 A1 sowie auch in der die Priorität der DE 103 10 722 beanspruchenden WO 2004/081136 A beschrieben. Als nachteilig hat sich bei diesem Aufbau jedoch herausgestellt, dass die Klebemasse um so stärker an Haftklebrigkeit verliert, je größer der Anteil der Klebemasse an elektrisch leitenden Bestandteilen ist, mittels derer eine Erwärmung der Klebemasse also überhaupt erst möglich wird. Darüber hinaus ergibt sich hierbei zum Teil die Schwierigkeit, mit den in der Regel amorphen haftklebrigen Polymeren überhaupt ein hinreichend ausgeprägtes kaltleitendes Verhalten auszubilden.

Auch bei diesem Aufbau besteht zusätzlich noch das Problem einer zu geringen Flexibilität des Flächenelements, infolge derer die Verlässlichkeit einer Klebeverbindung erheblich herabsetzt ist, was wiederum einen verringerten mechanischen, elektrischen und thermischen Kontakt über die Verklebung zur Folge hat, wodurch die maximal nutzbare Wärmemenge abnimmt und der Wärmeübergang erschwert wird.

Die nur für die Frage der Neuheit relevanten Schriften DE 10 2007 007 617 A sowie die aus dieser Schrift hervorgehende WO 2008/098847 A zeigen eine durchbrochene Kontaktierungsschicht auf einer erwärmbaren Heißschmelzklebemasse. Es ist jedoch kein Selbstklebeband offenbart, bei dem auf beiden Seiten eines durchbrochenen Kontaktierungselements jeweils Haftklebemassen angeordnet sind, die bei Raumtemperatur eine dauerhafte Verklebung mit einem Substrat eingehen.

Eine Aufgabe der vorliegenden Erfindung ist es daher, ein Flächenelement zur Verfügung zu stellen, das diese Nachteile beseitigt und das derart ausgebildet ist, dass es einen guten mechanischen und elektrischen Kontakt auch an einem Haftgrund mit gekrümmter Oberfläche ermöglicht, gleichzeitig an diesem eine hohe Verklebungsfestigkeit zeigt und zudem einen einfachen Aufbau aufweist, so dass dieses unter ökonomischen und ökologischen Gesichtspunkten günstig hergestellt werden kann. Insbesondere bestand eine Aufgabe darin, ein verbessertes intrinsisch erwärmbares und doppelseitig verklebendes Flächenelement zur Verfügung zu stellen, das eine erhöhte Flexibilität und/oder eine verbesserte Spaltüberbrückung sowie einen dünneren Aufbau bietet.

Diese Aufgabe wird überraschend und für den Fachmann in nicht vorhersehbarer Weise durch ein Flächenelement der eingangs genannten Art gelöst, bei dem die Kontaktierungsschicht ein zumindest im wesentlichen flächenförmig ausgebreitetes durchbrochenes Kontaktierungselement ist.

Gegenstand der Erfindung ist dementsprechend ein Flächenelement mit einer ersten selbstklebenden Seitenfläche und einer zweiten selbstklebenden Seitenfläche, wobei das Flächenelement eine Schichtabfolge aus einer Heizschicht, einer Kontaktierungsschicht und einer Klebemassenschicht aufweist, in der die Heizschicht mit einer ersten Seitenfläche der Kontaktierungsschicht in Kontakt steht und mit dieser elektrisch leitend verbunden ist, wobei sich die Heizschicht und die Kontaktierungsschicht unmittelbar berühren, und in der die Klebemasseschicht mit einer zweiten Seitenfläche der Kontaktierungsschicht in Kontakt steht und diese unmittelbar berührt, und wobei die Heizschicht aus einer intrinsisch erwärmbaren ersten Selbstklebemasse besteht, die als bei Hindurchleiten eines elektrischen Stroms sich erwärmender Kaltleiter ausgebildet ist, und die Klebemasseschicht aus einer zweiten Selbstklebemasse besteht, wobei die erste Selbstklebemasse und die zweite Selbstklebemasse jeweils Haftklebemassen sind, die bei Raumtemperatur eine dauerhafte Verklebung mit dem Substrat eingehen, und wobei die Kontaktierungsschicht ein zumindest im wesentlichen flächenförmig ausgebreitetes durchbrochenes Kontaktierungselement ist.

Durch die flächig-durchbrochene Ausbildung des Kontaktierungselements erhält dieses eine flexible Beschaffenheit und somit eine erhöhte Bruchfestigkeit. Dabei gewinnt das Kontaktierungselement parallel zu der Hauptausdehnung (Hauptausdehnungsebene) des Flächenelements an Flexibilität, so dass das Kontaktierungselement bei einer quer zur Hauptausdehnung einwirkenden Kraft beweglich-flexibel ist, ohne unter der so entstehenden mechanischen Spannung zu brechen. Gleichzeitig ist durch die zumindest im wesentlichen flächenförmige Ausdehnung des Kontaktierungselements sichergestellt, dass der Querschnitt des den elektrischen Strom leitenden Kontaktfläche mit der Heizschicht hinreichend groß ausfällt, um ein großflächiges Erwärmen sicherzustellen und so die Hauptfunktionalität zu gewährleisten.

Das erfindungsgemäße Flächenelement erfordert somit keine stabilisierende Trägerfolie, die dessen Flexibilität herabsetzt. Überraschend zeigt sich hierbei, dass das erfindungsgemäße Flächenelement eine höhere Verklebungsfestigkeit als entsprechende Aufbauten nach dem Stand der Technik aufweist. So wird durch die besondere flexible Ausbildung auch ohne zusätzliche Trägerfolie das Kriterium des Splitterschutzes erfüllt. Darüber hinaus kann bei einer Verwendung eines derartigen Flächenelements eine Verklebung erhalten werden, die eine Dicke aufweist, wie sie ansonsten lediglich bei Verwendung unbeheizter doppelseitiger Klebebänder erzielt wird, wodurch es möglich ist, einem dicken Gesamtaufbau entgegenzuwirken.

Darüber hinaus ist es günstig, wenn das Flächenelement zusätzlich zu den Merkmalen der vorgenannten Ausbildung derart ausgebildet ist, dass alle Teilbereiche des durchbrochenen Kontaktierungselements miteinander durch das durchbrochene Kontaktierungselement elektrisch leitend verbunden sind. Auf diese Weise ist das Kontaktierungselement als eine einzige Elektrode (Pol) der Heizschicht ausgebildet, so dass der Strom durch die gesamte Berührungsfläche (Kontaktfläche) des Kontaktierungselements mit der kaltleitenden Heizschicht hindurchtreten kann und so eine nahezu vollflächige Erwärmung des Flächenelements bewirken kann. Auf diese Weise ist es möglich, über eine maximale Fläche großflächig eine hohe Heizleistung zu erbringen. Die zweite Kontaktierung der Heizschicht (die andere Elektrode oder der andere Pol) erfolgt dann über ein weiteres Kontaktierungselement, das außerhalb des Flächenelements vorgesehen ist, beispielsweise eine den Strom gute leitende metallische Schicht an dem einen Haftgrund oder aber eine Metallschicht auf dem anderen Haftgrund.

Statt der vorgenannten Ausbildung kann es aber auch von Vorteil sein, wenn das durchbrochene Kontaktierungselement zumindest zwei Teilbereiche aufweist, die nicht miteinander über das durchbrochene Kontaktierungselement elektrisch leitend verbunden sind. Über derartige getrennte Teilbereiche, die beispielsweise als eine Vielzahl einzelner Teilabschnitte ausgebildet sein können, ist es möglich, beide elektrischen Kontakte (Elektroden oder Pole), die für eine Heizwirkung der Heizschicht erforderlich sind, innerhalb des Kontaktierungselements zu realisieren, so dass auf weitere den Strom leitende Schichten außerhalb des Flächenelements verzichtet werden kann und die Endmontage des Flächenelements an den Verklebungssubstraten somit stark vereinfacht wird. Darüber hinaus ist es auch möglich, an die einzelnen Teilabschnitte des Flächenelements jeweils unterschiedliche Spannungen anzulegen, etwa, um so einen Spannungsgradienten in der Fläche des Flächenelements zu erzeugen und die Heizleistung individuell den jeweiligen Bedürfnissen anzupassen.

Zusätzlich zu den Merkmalen einer oder mehrerer der vorgenannten Ausbildungen ist es darüber hinaus günstig, wenn das durchbrochene Kontaktierungselement Aussparungen aufweist, deren Hauptausdehnung zumindest im wesentlichen in einer Raumrichtung verläuft, der Vorzugsrichtung. Auf diese Weise lässt sich eine besonders hohe Flexibilität des Flächenelements in einer Richtung bei gleichzeitig minimaler Beeinträchtigung der mechanischen Stabilität und des elektrischen Kontaktquerschnitts erzielen. Dies kann etwa dann besonders sinnvoll sein, wenn das Flächenelement an einer zylindermantelartigen Fläche zu befestigen ist, die in einer Richtung stark gekrümmt ist und dabei einen kleinen Krümmungsradius aufweist.

Weiterhin kann es von Vorteil sein, wenn das durchbrochene Kontaktierungselement eine verzweigte Kammstruktur oder Fingerstruktur aufweist. Eine derartige Form ermöglicht eine optimale Nutzung nahezu der gesamten Fläche des Flächenelements zur Wärmeerzeugung bei nur kleinen Aussparungen, ohne dass hierdurch die mechanischen Eigenschaften in nennenswerter Weise beeinträchtigt werden oder ein starker Spannungsabfall über die Schicht zu erwarten ist. Bei einer Kammstruktur wie auch bei einer Fingerstruktur (Interdigitalstruktur) zweigen einzelne Zinken bzw. Finger von einem Hauptstrang ab. Der Hauptstrang kann hierbei einen größeren Querschnitt aufweisen als die Zinken bzw. Finger oder aber denselben Querschnitt. Der Unterschied zwischen einer Kammstruktur und einer Fingerstruktur besteht darin, dass die abzweigenden Elemente bei einer Kammstruktur auf derselben Seite vom Hauptstrang angeordnet sind, während bei einer Fingerstruktur diese auf unterschiedlichen Seiten abzweigen. Beide Strukturen können sowohl einfache als auch mehrfache Verzweigungen und dabei sowohl regelmäßige als auch unregelmäßige Anordnungen aufweisen und lassen sich sowohl bei einer Ausbildung des Kontaktierungselements als eine einzige Elektrode als auch bei einer Ausbildung des Kontaktierungselements als mehrere Elektroden innerhalb der Kontaktierungsschicht einsetzen.

Bei der ersten Selbstklebemasse ist es günstig, wenn diese zusätzlich zu den Merkmalen einer oder mehrerer der vorgenannten Ausbildungen mindestens einen elektrisch leitfähigen Füllstoff umfasst. Auf diese Weise lässt sich besonders einfach und kostengünstig eine kaltleitende Klebemasse erhalten, die eine für viele Anwendungen hinreichend hohe Heizleistung bietet. Besonders vorteilhaft ist es dabei, wenn der elektrisch leitende Füllstoff ausgewählt ist aus der Gruppe umfassend Graphit, Kohlenstoff-Nanoteilchen und Ruß, insbesondere Leitruß. Der Vorteil einer solchen Zusammensetzung besteht darin, dass diese Füllstoffe eine besonders gute Verbindung mit der Polymermatrix aufweisen, so dass eine derartige Klebemasse insgesamt über eine hohe Kohäsion verfügt und daher stark mechanisch belastbar wird.

Im Hinblick auf die kaltleitenden Eigenschaften der ersten Selbstklebemasse ist es dabei besonders günstig, wenn die erste Selbstklebemasse teilkristalline Polymere oder sogar teilkristalline Blockcopolymere aufweist, insbesondere zu einem Anteil von mehr als 30 Gew.-% in der ersten Selbstklebemasse, bevorzugt von mehr als 50 Gew.-%. Dies bietet den Vorteil, dass auf diese Weise als erste Selbstklebemasse Klebemassen eingesetzt werden können, die zusätzlich zu guten klebtechnischen Eigenschaften eine hohe Leitfähigkeit und gleichzeitig ein stark ausgeprägtes kaltleitendes Verhalten zeigen, so dass diese in besonderem Maße den Strom begrenzen können und daher einer Überhitzung effizient entgegenwirken.

Ferner hat es sich bei diesen Ausbildungen als günstig herausgestellt, wenn der elektrisch leitende Füllstoff in der ersten Selbstklebemasse zu einem Anteil von 1 Gew.-% bis 60 Gew.-% vorliegt, bevorzugt zu einem Anteil von 5 Gew.-% bis 30 Gew.-%, da sich auf diese Weise Klebemassen realisieren lassen, die einerseits eine hinreichend hohe Leitfähigkeit (damit überhaupt ein Strom durch die Selbstklebemasse fließt) und gleichzeitig eine hinreichend niedrige Leitfähigkeit aufweisen (damit die Wärmeentwicklung aufgrund des Spannungsabfalls an dem Widerstand nicht zu groß ist), so dass diese insgesamt als kaltleitende Heizmassen in Frage kommen, andererseits aber über einen hohen Klebstoffanteil verfügen, so dass zudem die Verklebungsfestigkeit gewährleistet ist.

Zusätzlich zu den Merkmalen einer oder mehrerer der vorgenannten Ausbildungen hat es sich als günstig herausgestellt, wenn die erste Selbstklebemasse und/oder die zweite Selbstklebemasse eine Haftklebemasse ist bzw. sind. Derartige Systeme ermöglichen ein besonders einfaches Verkleben, ohne dass hierfür weitere Prozessschritte wie etwa ein Erhitzen der Flächenelemente erforderlich ist, so dass derartige Selbstklebemassen auch bei einem Haftgrund eingesetzt werden können, der eine stark unregelmäßige Geometrie aufweist oder wärmeempfindlich ist.

Als Haftklebemassen haben sich insbesondere solche als vorteilhaft herausgestellt, die zumindest teilweise auf mindestens einem Acrylmonomer der allgemeinen Formel $CH_2=C(R^1)(COOR^2)$ basieren, wobei $R^1$ ausgewählt ist aus der Gruppe umfassend H und $CH_3$ und $R^2$ ausgewählt ist aus der Gruppe umfassend H sowie gesättigte oder ungesättigte, unverzweigte oder verzweigte, substituierte oder unsubstituierte $C_1$- bis $C_{30}$-Alkylreste, insbesondere, dass die Haftklebemasse zumin-

9 EP 2 148 337 B1 10

dest teilweise auf mindestens einem Acrylmonomer der allgemeinen Formel $CH_2=C(R^1)(COOR^{2'})$ basiert, wobei $R^1$ ausgewählt ist aus der Gruppe umfassend H und $CH_3$ und $R^{2'}$ ausgewählt ist aus der Gruppe umfassend H und gesättigte, unverzweigte oder verzweigte, substituierte oder unsubstituierte $C_2$- bis $C_{20}$-Alkylreste, sowie ferner zumindest teilweise auf einem mit diesem mindestens einen Acrylmonomer polymerisierbaren Comonomer basiert, das insbesondere gewählt wird aus der Gruppe umfassend Vinylverbindungen mit funktionellen Gruppen, Maleinsäureanhydrid, Styrol, Styrol-Verbindungen, Vinylacetat, Acrylamide oder mit Doppelbindungen funktionalisierte Photoinitiatoren. Stattdessen kann die Haftklebemasse aber auch zumindest teilweise eine Naturkautschukmasse und/oder eine Synthesekautschukmasse sowie Silikonklebemasse umfassen. Derartige Haftklebemassen bieten den Vorteil, dass sich die klebtechnischen Eigenschaften des Flächenelements im breiten Umfang steuern lassen und somit gezielt auf die konkreten Verhältnisse der zu erzielenden Verklebung eingestellt werden können, etwa im Hinblick auf den jeweiligen Haftgrund oder die Umgebungsbedingungen.

Zusätzlich zu den Merkmalen einer oder mehrerer der vorgenannten Ausbildungen hat es sich als günstig herausgestellt, wenn die Zusammensetzung der ersten Selbstklebemasse mit der Zusammensetzung der zweiten Selbstklebemasse identisch ist. Auf diese Weise kann auf äußerst einfache Weise ein intrinsisch erwärmbares Flächenelement mit besonders hoher Heizleistung realisiert werden. Stattdessen kann es aber auch sinnvoll sein, wenn die Zusammensetzung der ersten Selbstklebemasse von der Zusammensetzung der zweiten Selbstklebemasse verschieden ist, wodurch es insbesondere möglich ist, zwei Verklebungssubstrate unterschiedlicher Beschaffenheit miteinander zu verkleben, etwa eine polare Glasoberfläche mit einer unpolaren Polymeroberfläche, etwa einem Polyolefin.

Zusätzlich zu den Merkmalen einer oder mehrerer der vorgenannten Ausbildungen hat es sich als günstig herausgestellt, wenn das Flächenelement an der dem durchbrochenen Kontaktierungselement abgewandten Seitenfläche der Heizschicht eine dritte Selbstklebemasse aufweist. Hierdurch können die klebtechnischen Eigenschaften der ersten Selbstklebemasse von denen der selbstklebenden Seitenflächen des Flächenelements entkoppelt werden. Somit ist es möglich, eine besonders hohe Verklebungsstabilität insgesamt zu erreichen, da die Klebemassen an den selbstklebenden Seitenflächen des Flächenelements individuell auf den jeweiligen Klebegrund eingestellt werden können und gleichzeitig die Heizschicht für eine besonders gute Verankerung an dem Kontaktierungselement angepasst werden kann, was insbesondere dann von Bedeutung ist, wenn das Material der Oberfläche des Kontaktierungselements stark von dem Material der Oberfläche des Klebegrunds abweicht, etwa im Falle eines metallischen Kontaktierungselements und eines polyolefinischen Klebegrunds.

Bei dem Flächenelement kann zusätzlich zu den Merkmalen einer oder mehrerer der vorgenannten Ausbildungen die Heizschicht eine Dicke von weniger als 1 mm aufweisen, bevorzugt eine Dicke aus einem Bereich von 10 $\mu$m bis 400 $\mu$m, besonders bevorzugt aus einem Bereich von 20 $\mu$m bis 200 $\mu$m. Eine derartige Ausgestaltung der Heizschicht stellt optimale Eigenschaften sicher, da die Heizschicht einerseits dick genug ist, um eine hinreichend hohe Heizleistung zur Verfügung zu stellen, andererseits dünn genug, um eine schnelle Wärmeleitung innerhalb der Heizschicht sowie gute mechanische Eigenschaften im Hinblick auf Flexibilität und Kohäsion zu gewährleisten.

Ferner ist es von Vorteil, wenn das Flächenelement zusätzlich zu den Merkmalen einer oder mehrerer der vorgenannten Ausbildungen einen flexiblen permanenten Träger umfasst. Hierdurch wird ein besonders stabiles Flächenelement mit einem hohen Maß an Flexibilität erhalten. Anstelle dessen kann das Flächenelement allerdings auch trägerfrei ausgebildet sein. Dies ist besonders dann günstig, wenn eine besonders hohe Flexibilität und/oder eine geringe Einbautiefe des Flächenelements erforderlich ist.

Im Hinblick auf die Handhabbarkeit hat es sich außerdem als sinnvoll herausgestellt, wenn das Flächenelement zusätzlich zu den Merkmalen einer oder mehrerer der vorgenannten Ausbildungen an seiner ersten selbstklebenden Seitenfläche und/oder an seiner zweiten selbstklebenden Seitenfläche einen temporären Träger aufweist. Hierdurch ist es möglich, während der Herstellung, Lagerung und Verklebung ein unbeabsichtigtes Verkleben zu vermeiden und diese Schritte somit einfacher zu gestalten.

Gemäß einem weiteren Aspekt der vorliegenden Erfindung wird ein Verklebungsverbund aus einem Verklebungssubstrat und einem der vorgenannten Flächenelemente vorgeschlagen. Die bisher bekannten Verklebungsverbunde haben den Nachteil, dass diese auf gekrümmten (gebogenen) Oberflächen nicht zuverlässig dauerhaft verklebt werden können, da es infolge der Eigensteifigkeit der einseitig damit verklebten selbstklebenden Flächenelemente zu einem Ablösen von der gekrümmten Oberfläche kommen kann. Dieser Nachteil wird durch Verwendung des erfindungsgemäßen Flächenelements vermieden. Insbesondere ist dies günstig, wenn es sich bei dem Verklebungsverbund um einen Verbund aus zumindest einem doppelseitig selbstklebenden Flächenelement und einer Sichtscheibe oder Spiegelscheibe als Verklebungssubstrat handelt, da bei derartigen Systemen infolge des hohen Eigengewichts des Verklebungssubstrats ein Ablösen des Verklebungssubstrats aus der Halterung und die daraus potentiell resultierende Zerstörung des Verklebungssubstrats besonders problematisch ist.

Ferner schlägt die vorliegende Erfindung die Verwendung des zuvor beschriebenen Flächenelements zur Verklebung von Verklebungssubstraten in der Automobilindustrie vor, insbesondere zum Beheizen des vor-

6

genannten Verklebungsverbunds. Wird ein auf einem Verklebungssubstrat mit gekrümmter Oberfläche verklebter Verklebungsverbund der bisher bekannten Art intrinsisch erwärmt, so geht mit der Erwärmung der Klebemasse eine Erweichung der Klebemasse und damit auch eine Abnahme der Kohäsion der Klebemasse einher. Dies kann infolge der hohen Eigensteifigkeit der bisher verwendeten Flächenelemente ein Aufspalten der erweichten Klebemasse zur Folge haben, so dass sich der Verklebungsverbund vom Verklebungssubstrat ablöst. Dieser Nachteil wird durch Verwenden des erfindungsgemäßen Flächenelements zum Beheizen des Verklebungsverbunds vermieden.

Darüber hinaus ist erfindungsgemäß auch eine Verwendung möglich, bei der ein derartiges Flächenelement auf der Oberfläche eines menschlichen oder tierischen Körpers verklebt wird, beispielsweise zur Freisetzung eines verkapselten Wirkstoffs an die Haut oder an der Felloberseite, etwa in Form eines topisch wirksamen oder transdermalen Pflasters. Dabei weist das Flächenelement zumindest einen aktiven Stoff auf, der durch Wärme freisetzbar ist oder dessen Freisetzung durch Wärme unterstützt wird. Auf diese Weise kann in besonders einfacher Weise eine zeitlich und mengenmäßig kontrollierte Freisetzung der entsprechenden aktiven Stoffe erfolgen.

Schließlich stellt die vorliegende Erfindung ein Verfahren zur Herstellung eines Flächenelements bereit, das die Schritte eines Ausbildens einer ersten Klebeschichtung, eines Auftragens des durchbrochenen Kontaktierungselements direkt auf die Oberfläche der ersten Klebeschichtung, und eines Auftragens einer zweiten Klebeschichtung auf die Oberfläche des durchbrochenen Kontaktierungselements umfasst. Bei den bisherigen Verfahren wird das Kontaktierungselement stets an einem permanenten Träger eingesetzt, so dass damit lediglich dicke Flächenelemente mit in der Regel nur geringer Flexibilität erhalten werden. Infolge des Aufbringens des Kontaktierungselements direkt auf die Oberfläche einer Klebemasse können mit geringem prozesstechnischem Aufwand Flächenelemente geringer Dicke erhalten werden, ohne dass hierfür das Kontaktierungselement gesondert auf einen Träger (permanent oder temporär) aufgetragen werden muss, was eine Vereinfachung des Herstellungsverfahrens insgesamt zur Folge hat.

Die einzelnen vorteilhaften Ausgestaltungen lassen sich, soweit nicht anders angegeben, beliebig untereinander kombinieren und damit die zuvor beschriebenen sowie weitere vorteilhafte Wirkungen erzielen; diese Merkmale werden daher auch für sich genommen in Kombination mit den Merkmalen der unabhängigen Ansprüche als schutzfähig erachtet.

Zur Verdeutlichung der Erfindung ist im Folgenden eine allgemeine Beschreibung der Erfindung gegeben, wozu einige repräsentative Beispiele einzelner Bestandteile von Teilaspekten der Erfindung beschrieben sind, die in Abhängigkeit von den jeweils gewünschten Eigenschaften nahezu beliebig miteinander verknüpft werden können. Grundsätzlich betrifft die Erfindung ein Flächenelement mit einer ersten selbstklebenden Seitenfläche und einer zweiten selbstklebenden Seitenfläche. Als Flächenelement im Sinne dieser Anmeldung gelten insbesondere alle üblichen und geeigneten Gebilde mit im wesentlichen flächenförmiger Ausdehnung. Diese ermöglichen eine Verklebung und können dabei verschieden ausgestaltet sein, insbesondere flexibel, als Klebefolie, Klebeband, Klebeetikett oder als Formstanzling.

Dieses Flächenelement ist an seiner ersten Oberfläche und auch an seiner zweiten Oberfläche selbstklebend ausgebildet. Hierbei entspricht die erste und die zweite Oberfläche jeweils den beiden Oberflächen des Flächenelements parallel zu seiner Hauptausdehnung, also dessen Oberseite und Unterseite.

Als selbstklebend im Sinne dieser Anmeldung wird insbesondere eine Oberfläche bezeichnet, die an dieser zumindest lokal, bevorzugt aber großflächig oder sogar vollflächig eine Selbstklebemasse aufweist. Unter Selbstklebemassen werden vorliegend ausnahmslos alle Klebemassen auf der Basis von Haftklebemassen und/oder Heißschmelzklebemassen verstanden, also Klebemassen, die von sich aus eine dauerhafte Verklebung mit dem Substrat (dem Untergrund, Haftgrund oder Klebegrund) erlauben. "Auf der Basis von" oder "auf der Grundlage von" bedeutet vorliegend, dass die klebtechnischen Eigenschaften dieses Klebemassesystems zumindest stark von den grundlegenden Eigenschaften dieser Klebemasse oder Klebemassenbestandteile (dem so genannten Basispolymer) bestimmt werden, wobei selbstverständlich nicht ausgeschlossen ist, dass diese durch Verwendung von modifizierenden Hilfs- oder Zusatzstoffen oder von weiteren polymeren Klebemassen in dem Klebesystem zusätzlich beeinflusst werden.

Als Haftklebemassen werden solche Klebemassen bezeichnet, die bei Raumtemperatur bereits unter relativ schwachem Andruck eine dauerhafte Verklebung mit dem Substrat erlauben. Im Gegensatz dazu werden als Heißschmelzklebemassen solche Klebemassen bezeichnet, die erst bei erhöhten Temperaturen eine dauerhafte Verklebung mit dem Substrat eingehen, wobei die so erhaltene Verklebung auch bei einem anschließenden Abkühlen der Verklebung auf Raumtemperatur noch erhalten bleibt. Die Verklebbarkeit von Haftklebemassen wie auch von Heißschmelzklebemassen beruht auf ihren adhäsiven Eigenschaften.

Als Adhäsion wird üblicherweise der physikalische Effekt bezeichnet, der den Zusammenhalt zweier miteinander in Kontakt gebrachter Phasen an ihrer Grenzfläche aufgrund dort auftretender intermolekularer Wechselwirkungen bewirkt. Die Adhäsion bestimmt somit das Anhaften der Klebemasse an der Substratoberfläche und ist als Anfassklebrigkeit (dem so genannten Tack) und als Klebkraft bestimmbar. Um die Adhäsion einer Klebemasse gezielt zu beeinflussen, werden der Klebemasse häufig Weichmacher und/oder klebkraftsteigernde Harze (so genannte Tackifier) zugesetzt.

Als Kohäsion bezeichnet man üblicherweise den physikalischen Effekt, der den inneren Zusammenhalt eines Stoffs oder Stoffgemisches aufgrund intermolekularer und/oder intramolekularer Wechselwirkungen zur Folge hat. Die Kohäsionskräfte bestimmen somit die Zähflüssigkeit und Fließfähigkeit der Klebemasse, die sich etwa als Viskosität und als Scherstandzeit bestimmen lassen. Um die Kohäsion einer Klebemasse gezielt zu erhöhen, werden diese häufig einer zusätzlichen Vernetzung unterzogen, wofür der Klebemasse reaktive (und somit vernetzbare) Bestandteile oder andere chemische Vernetzer zugesetzt werden und/oder die Klebemasse in einer Nachbehandlung aktinischen (energiereichen) Strahlen ausgesetzt wird.

Die klebtechnischen Eigenschaften einer Haftklebemasse werden in erster Linie von dem Verhältnis adhäsiver und kohäsiver Eigenschaften bestimmt. So ist es beispielsweise für einige Anwendungen wichtig, dass die eingesetzten Klebemassen hochkohäsiv sind, also über einen besonders starken inneren Zusammenhalt verfügen, während für andere Anwendungen eine besonders hohe Adhäsion erforderlich ist.

Erfindungsgemäß weist das Flächenelement eine bestimmte Schichtabfolge auf. Als Schichtabfolge wird insbesondere eine räumliche Anordnung von einzelnen Schichten bezeichnet, die senkrecht zu ihrer Hauptausdehnung übereinander (stapelförmig) angeordnet sind und jeweils direkt miteinander in Kontakt stehen, ohne dass dazwischen weitere Schichten vorhanden sind. Als Schicht wird insbesondere eine flächenförmige Anordnung eines Systems einheitlicher Funktionalität bezeichnet, deren Abmessungen in einer Raumrichtung signifikant kleiner ist als in den beiden anderen Raumrichtungen, die die Hauptausdehnung definieren. Eine derartige Schicht kann kompakt oder auch durchbrochen ausgebildet sein und dabei aus einem Material oder aus unterschiedlichen Materialien bestehen, insbesondere, wenn diese zur einheitlichen Funktionalität dieser Schicht beitragen. Diese Schicht kann eine über ihre gesamte Flächenausdehnung konstante Dicke aufweisen oder aber unterschiedliche Dicken. Darüber hinaus kann eine Schicht natürlich auch mehr als eine einzige Funktionalität aufweisen.

Innerhalb der erfindungsgemäßen Schichtabfolge ist eine Kontaktierungsschicht zwischen einer Heizschicht und einer Klebemassenschicht angeordnet. Als Kontaktierungsschicht wird jede den elektrischen Strom gut leitende Schicht bezeichnet, mit deren Hilfe eine Spannung an die Heizschicht angelegt und/oder ein Strom durch die Heizschicht hindurch geleitet werden kann; die Kontaktierungsschicht dient somit zum Anschluss externer elektrischer Versorgungsleitungen an das Flächenelement (Kontaktierungselektrodenfunktion). Als Heizschicht wird jede Schicht bezeichnet, die zur Erwärmung des Flächenelements eingerichtet ist. Als Klebemassenschicht wird jede Schicht bezeichnet, die eine Klebemasse enthält und zum klebenden Verbinden des Flächenelements mit einem Haftgrund angepasst ist.

Demzufolge steht die Heizschicht mit einer ersten Seitenfläche der Kontaktierungsschicht (also der Oberseite oder der Unterseite der Kontaktierungsschicht) in Kontakt, so dass sich diese beiden Schichten direkt, das heißt unmittelbar, berühren. Zudem ist die Heizschicht mit der ersten Seitenfläche der Kontaktierungsschicht elektrisch leitend verbunden. Eine Verbindung wird insbesondere dann als elektrisch leitend bezeichnet, wenn der elektrische Gesamtwiderstand der Verbindung, der sich aus den Widerständen der zu verbindenden Teilabschnitte und dem Kontaktwiderstand der Verbindung zusammensetzt, höchstens von derselben Größenordnung ist wie die Gesamtwiderstände der restlichen leitenden Bereiche und Kontakte.

Darüber hinaus steht die zweite Seitenfläche der Kontaktierungsschicht (entsprechend der anderen Seitenfläche, also die Unterseite oder die Oberseite der Kontaktierungsschicht) mit der Klebemasseschicht in Kontakt und berührt diese unmittelbar. Die Klebemasseschicht besteht hierbei aus einer zweiten Selbstklebemasse. Als Selbstklebemassen kommen alle üblichen und geeigneten Haftklebemassen in Frage.

Die Heizschicht besteht erfindungsgemäß zudem aus einer intrinsisch erwärmbaren ersten Selbstklebemasse. Auch für die erste Selbstklebemasse kommen alle üblichen und geeigneten Haftklebemassen in Frage. Als intrinsisch erwärmbare Schicht gilt jede Schicht, die von sich aus elektrisch erwärmbar ist, das heißt, dass diese Schicht ohne weitere Bauteile oder Komponenten in der Schicht in der Lage ist, bei Hindurchleiten eines elektrischen Stroms durch die Schicht oder bei Anlegen einer elektrischen Spannung an die Schicht selber Wärme zu produzieren, wobei es unerheblich ist, ob der Strom bzw. die Spannung ein Wechselstrom bzw. eine Wechselspannung ist oder aber ein Gleichstrom bzw. eine Gleichspannung. Überdies ist die erste Selbstklebemasse als Kaltleiter ausgebildet, der sich bei Hindurchleiten eines elektrischen Stroms erwärmt.

Als ein erfindungswesentliches Element ist die Kontaktierungsschicht ein zumindest im wesentlichen flächenförmig ausgebreitetes durchbrochenes Kontaktierungselement. Als Kontaktierungselement wird insbesondere ein Element aus einem den elektrischen Strom leitenden Material bezeichnet, dessen Struktur zumindest in einem Teilbereich den Strom durchgängig elektrisch leitet. "Zumindest im wesentlichen flächenförmig ausgebreitet" bedeutet, dass die Teilbereiche, aus denen sich die Kontaktierungsschicht zusammensetzt, innerhalb der Schicht in einer flächigen Anordnung vorliegen, wobei einzelne Teilbereiche auch aus dieser flächigen Anordnung herausragen können.

Die Kontaktierungsschicht ist eine elektrisch leitende Verbindung zwischen der Heizschicht und der Stromquelle bzw. Spannungsquelle. Hierbei kann die Kontaktierungsschicht entweder als einer der beiden Elektrodenanschlüsse (Pole) der Heizschicht ausgebildet sein oder aber beide Elektrodenanschlüsse bilden.

Stellt die Kontaktierungsschicht lediglich einen der beiden Elektrodenanschlüsse der Heizschicht dar, so ist ein zweiter Elektrodenanschluss erforderlich, damit durch die Heizschicht ein Strom fließen kann und die Heizschicht sich erwärmt. Dieser zweite Elektrodenanschluss kann dabei innerhalb des erfindungsgemäßen Flächenelements ausgebildet sein - etwa in Form einer zusätzlichen zweiten flexiblen Kontaktierungsschicht - oder aber an einem der beiden Verklebungssubstrate vorgesehen sein, beispielsweise als metallische Schicht an der Oberfläche eines Glases (etwa als Silberschicht eines Spiegels).

Erfindungsgemäß ist die Kontaktierungsschicht nicht vollflächig geschlossen sondern vielmehr durchbrochen, so dass diese diskontinuierliche Schicht Aussparungen (Vertiefungen) aufweist, die sich auch in einer Richtung senkrecht zur Hauptausdehnung der Schicht erstrecken. Demzufolge ist die Schicht selber nicht vollflächig kompakt, sondern weist Aussparungen auf, die sich durch die Schicht hindurch erstrecken können (durchgängige Löcher) oder aber lediglich auf Teile der Schicht beschränkt sind (Mulden, etwa zur lokalen Verringerung der Dicke der Kontaktierungsschicht). Die Vertiefungen können dabei beliebig geformt sein, etwa regelmäßig oder unregelmäßig, von gleichmäßiger oder wechselnder Breite sein, gerade, schräge oder kurvenförmige Wandabschnitte aufweisen und dergleichen. Die Vertiefungen können daher in der Kontaktierungsschicht in unterschiedliche Richtungen verlaufen oder aber eine besondere Vorzugsrichtung aufweisen, so dass die Kontaktierungsschicht innerhalb ihrer Hauptausdehnung quer zu dieser Vorzugsrichtung eine besonders hohe Flexibilität aufweist.

Im Hinblick auf die konkrete Ausgestaltung dieser Vertiefungen kann das Kontaktierungselement unterschiedlich gestaltet sein, etwa als unterbrochene Fläche, als aufgefaltete oder verzweigte Drahtstruktur oder dergleichen, beispielsweise als einfach oder mehrfach verzweigte Kammstruktur oder Fingerstruktur. Weitere geeignete Kontaktierungselemente sind etwa durchbrochene Metallfolien, Streckmetallgitter, Drahtgitter, Metallnetze oder elektrisch leitende Vliese. Auch nichtmetallische Leiter wie Metalloxide (beispielsweise Indium-Zinn-Oxid) oder intrinsisch leitfähige Polymere sind erfindungsgemäß einsetzbar. Zur Verbesserung der Flexibilität des Flächenelements hat die zumindest eine Kontaktierungsschicht bevorzugt eine mittlere oder sogar maximale Dicke von weniger als 50 $\mu$m, bevorzugt von weniger als 20 $\mu$m oder sogar von weniger als 10 $\mu$m.

Die den elektrischen Strom leitenden Bereiche des Kontaktierungselements können miteinander leitend verbunden sein (alle Bereiche oder nur ein Teil der Bereiche) oder jeweils als einzelner Bereich des Kontaktierungselements vorliegen, der nicht mit den anderen Bereichen über das Kontaktierungselement leitend verbunden ist. Gegebenenfalls kann auch ein Teil der Bereiche elektrisch leitend miteinander verbunden sein und ein anderer Teil der Bereiche jeweils einzeln vorliegen. Dies schließt selbstverständlich eine leitende Verbindung über die Heizschicht nicht aus, die erfindungsgemäß sogar erforderlich ist.

Insbesondere ist vorgesehen, dass das Kontaktierungselement zwei nicht miteinander leitend verbundene Bereiche enthält, die als die zwei Elektrodenzuleitungen (Pole) der Heizschicht ausgebildet sind. Ist das gesamte Kontaktierungselement durchgängig leitend, so wird dies als einer der beiden Pole der Heizschicht verwendet, wobei ein Stromfluss durch die Heizschicht dann hauptsächlich in einer Richtung senkrecht zur Hauptausdehnung auftritt, wohingegen bei einer Anordnung, bei der das Kontaktierungselement beide Elektrodenanschlüsse darstellt, zusätzlich zu dem senkrechten Stromfluss oder anstelle dessen ein lateraler Stromfluss innerhalb der Hauptausdehnung auftritt.

Als erste Selbstklebemasse der Heizschicht können grundsätzlich alle Haftklebemassen verwendet werden, die einen durch diese Selbstklebemasse fließenden elektrischen Strom im wesentlichen zersetzungsfrei leiten. Bevorzugt wird die Wärme innerhalb der selbstklebenden Heizschicht aus dem infolge des elektrischen Widerstands auftretenden Spannungsabfall in dieser Schicht selbst erzeugt, jedoch kann ein Heizen auch auf der Grundlage anderer Effekte erreicht werden, etwa mittels eines anderen elektrothermischen Wandlers oder einer elektrisch initiierten exothermen chemischen Reaktion. Erfindungsgemäß können solche Flächenelemente einmal oder mehrfach einsetzbar ausgebildet sein; ebenfalls kann der Wärmeerzeugungsprozess einmalig oder mehrfach durchführbar sein. Eine derartige Heizschicht kann senkrecht zur Hauptausdehnung eine (mittlere) Dicke von weniger als 1 mm aufweisen, bevorzugt eine Dicke aus einem Bereich von 10 $\mu$m bis 400 $\mu$m, besonders bevorzugt aus einem Bereich von 20 $\mu$m bis 200 $\mu$m. Für den bevorzugten Fall einer als Heizwiderstand eingesetzten Schicht kann diese einen elektrischen Widerstand aufweisen, der einerseits hoch genug ist, um eine Erwärmung der Schicht zu ermöglichen, andererseits niedrig genug ist, um einen Stromfluss durch die Schicht überhaupt zu etablieren.

Erfindungsgemäß muss diese Heizschicht außerdem kaltleitend sein, das heißt einen positiven Temperaturkoeffizienten aufweisen und somit einen PTC-Effekt zeigen. Bevorzugt ist die Schicht hinsichtlich ihres positiven Temperaturkoeffizienten und des Widerstands so ausgelegt, dass für die jeweilige Betriebsspannung und den jeweiligen Betriebsstrom die Wärmeerzeugung in der Heizschicht durch den PTC-Effekt begrenzt wird, so dass sich die Schicht bezüglich der Wärmeentwicklung selbstregulierend verhält und insbesondere einen festgelegten Temperaturhöchstwert nicht überschreitet. Eine Überhitzung des Flächenelements kann somit vermieden werden.

Bevorzugt wird hierbei als erste Selbstklebemasse eine Haftklebemasse eingesetzt, die mindestens einen elektrisch leitfähigen Füllstoff als elektrisch leitfähiges Material umfasst. Als elektrisch leitfähiger Füllstoff wird eine Beimengung zu einer Selbstklebemasse angesehen, die elektrischen Strom entweder bereits für sich alleine (also ohne Selbstklebemasse) leitet oder auch erst in der Mischung mit der Selbstklebemasse.

Als Füllstoff sind grundsätzlich alle geeigneten Füllstoffe einsetzbar, die mit der jeweiligen ersten Selbstklebemasse kompatibel sind. Insbesondere werden hierfür Füllstoffe eingesetzt, die ausgewählt sind aus der Gruppe umfassend Graphit und Ruß, insbesondere Leitruß (beispielsweise Printex® XE der Fa. Degussa), sowie beliebige Kombinationen davon. Zusätzlich oder stattdessen können bevorzugt auch andere Füllstoffe auf Kohlenstoffbasis eingesetzt werden, insbesondere solche, die nanoskalig sind, also in mindestens einer Raumdimension eine Ausdehnung von nicht mehr als 500 nm, bevorzugt von weniger als 200 nm oder sogar von weniger als 50 nm aufweisen, zum Beispiel Kohlenstoff-Nanoteilchen wie Kohlenstoff-Nanoröhren (beispielsweise Carbon Nanotubes der Fa. Ahwahnee oder Carbon-Nanotube-Masterbatches der Fa. Hyperion Catalysis), Kohlenstoff-Nanofasern (carbon nanofibres), Fullerene und dergleichen.

Vorteilhafterweise wird der Füllstoff in einer Menge eingesetzt, so dass der Anteil des Füllstoffs in der ersten Selbstklebemasse groß genug ist, um eine hinreichende Leitfähigkeit der ersten Selbstklebemasse zu gewährleisten, andererseits aber gering genug ist, um die mechanischen Eigenschaften der ersten Selbstklebemasse nur wenig zu beeinträchtigen. Vorteilhaft kann darüber hinaus auch eine Kombination von verschiedenartigen Füllstoffen sein, da hiermit ausreichende Kaltleiteigenschaften bei einem möglichst geringen Füllgrad erreicht werden können, insbesondere bei der Kombination von Kohlenstoff-Nanoröhren mit Ruß oder Graphit.

Weiterhin können die Füllstoffe oberflächenmodifiziert eingesetzt werden. Dadurch kann gezielt auf einzelne Eigenschaften der ersten Selbstklebemasse Einfluss genommen werden, etwa, um die Dispergierbarkeit von Kohlenstoff-Nanoröhren oder Ruß in der Selbstklebemasse zu verbessern. Zur Steigerung des PTC-Effekts kann die Oberfläche der leitfähigen Füllstoffe, etwa der Rußpartikel, vollständig oder teilweise mit Metallen wie Nickel, Silber oder Gold, mit Silanen oder mit Formamiden bedeckt werden.

Die Leitfähigkeit und somit auch die erzielbare Temperatur und Aufheizrate ist unter anderem abhängig vom Füllgrad des leitfähigen Füllstoffs, das heißt dessen Massenanteil in der Selbstklebemasse. Durch Anheben des Füllgrades lassen sich höhere Leitfähigkeiten und gegebenenfalls auch höhere Temperaturen erreichen. Somit kann die Ausprägung des Effektes der elektrischen Erwärmbarkeit der ersten Selbstklebemasse durch den Füllgrad bestimmt werden. Der Füllgrad beträgt vorteilhafterweise zwischen 1 und 60 Gew.-%. Sehr bevorzugt werden zwischen 5 und 30 Gew.-% an Füllstoff eingesetzt. Die elektrische Leitfähigkeit und somit die Erwärmbarkeit der ersten Selbstklebemasse ist ferner auch von deren Basispolymer abhängig.

Um die kaltleitende erste Selbstklebemasse zu erhalten, können die elektrisch leitfähigen Füllstoffe den Monomeren der ersten Selbstklebemasse vor der Polymerisation und/oder während der Polymerisation beigemischt werden und/oder mit den Polymeren erst nach Beendigung der Polymerisation vermengt werden. Vorzugsweise wird der leitfähige Füllstoff nach der Polymerisation zu einer Schmelze eines Basispolymers der ersten Selbstklebemasse hinzugegeben.

Insbesondere wenn die erste Selbstklebemasse auf das erfindungsgemäße Flächenelement als Hotmelt-System aus der Schmelze aufgetragen wird, wird der elektrisch leitfähige Füllstoff bevorzugt direkt in die Schmelze eingebracht. Hierbei ist eine homogene Einarbeitung im erfindungsgemäßen Sinne wünschenswert. Homogene Verteilungen des Füllstoffs in der ersten Selbstklebemasse werden bevorzugt durch eine Kompoundierung in Doppelschneckenextrudern, kontinuierlichen Knetern (beispielsweise Buss-Kneter) oder Planetwalzenextrudern erreicht. Ein Vorteil dieses Prozesses ist eine nur kurzzeitige Kontaminierung des Herstellungsprozesses mit dem separaten Füllstoff sowie die Vermeidung von Lösungsmitteln.

Grundsätzlich lassen sich in der Heizschicht als erste Selbstklebemasse alle Polymere mit geeigneten klebenden Eigenschaften einsetzen, die einen PTC-Effekt aufweisen, sich also kaltleitend verhalten. Dabei hängt das Auftreten und das Ausmaß eines PTC-Effekts von einer Netzwerkausbildung ab, beispielsweise davon, ob der leitfähige Füllstoff selber agglomeriert vorliegt oder nicht. Der PTC-Effekt lässt sich dabei unter anderem durch während des Herstellprozesses eingebrachte Orientierungen innerhalb der polymeren Bestandteile der ersten Selbstklebemasse unterstützen, etwa, indem eine Anisotropie bezüglich physikalischer Eigenschaften und/oder bezüglich der Ausrichtung der Makromoleküle eingebracht wird.

Wird als kaltleitendes System eine Selbstklebemasse mit einem elektrisch leitfähigen Füllstoff eingesetzt, dann hat es sich als vorteilhaft herausgestellt, mehrphasige Systeme zu verwenden, insbesondere solche, bei denen zumindest eine Phase in dem Temperaturbereich, in dem der PTC-Effekt auftritt, infolge der Erwärmung eine Volumenausdehnung erfährt, die nach allgemein anerkannter wissenschaftlicher Erklärung zumindest teilweise für das kaltleitende Verhalten verantwortlich ist (siehe J. Meyer in Polymer Engineering and Science, 13 (1973), S. 462 - 468). Als mehrphasig im Sinne der Erfindung werden auch Selbstklebemassen auf der Basis von Polymeren oder Polymermischungen (so genannte Polymerblends) aufgefasst, die zusätzlich zu dem leitfähigen Füllstoff einen oder mehrere weitere Füllstoffe aufweisen.

Als besonders vorteilhaft hat sich hierbei der Einsatz von solchen Selbstklebemassen herausgestellt, die teilkristalline Polymere aufweisen. Als teilkristalline Polymersysteme können sowohl einphasige als auch mehrphasige Systeme eingesetzt werden, sowohl Homopolymere als auch Copolymere, insbesondere teilkristalline Blockcopolymere. Die teilkristallinen Polymere können dabei ein Teil des Basispolymers selbst sein oder aber einen Zuschlagstoff darstellen. Die kristallinen Teilbereiche derartiger teilkristalliner Polymere weisen beim Erweichen der Polymermatrix eine stärkere Wärmeausdehnung als dessen amorphe Bereiche.

Bevorzugt enthält die die Haftklebemasse in der Heizschicht mindestens 30 Gew.-% teilkristalliner Polymere, noch besser ist ein Anteil an teilkristallinen Polymeren von mindestens 50 Gew.-% in der Selbstklebemasse. Haftklebemassen verlieren mit zunehmendem teilkristallinen Anteil ihre haftklebrigen Eigenschaften, so dass bei Verwendung von Haftklebemassen der Anteil an teilkristallinen Polymeren niedriger zu halten ist als bei Heißschmelzklebemassen, um eine noch hinreichend hohe Haftklebrigkeit zu gewährleisten.

In einer Haftklebemasse als erster Selbstklebemasse sind insbesondere solche teilkristallinen Polymere vorteilhaft, bei denen der Kristallinitätsgrad mehr als 20 % oder sogar mehr als 40 % beträgt. Der Kristallinitätsgrad lässt sich mit Hilfe der dynamischen Differenzkatorimetrie (Differential Scanning Calorimetry; DSC) ermitteln.

Ganz besonders bevorzugt werden als Blockcopolymere Styrol-Blockcopolymere wie etwa SBS (Styrol-Butadien-Styrol-Blockcopolymere), SIS (Styrol-Isopren-Styrol-Blockcopolymere), SEBS (Styrol-Ethylen-Butylen-Styrol-Blockcopolymere) oder SEPS (Styrol-Ethylen-Propylen-Styrol-Blockcopolymere) eingesetzt.

Vorteilhaft ist auch der Zusatz polymerer oder anorganischer Füllstoffe, die während der Erwärmung mit ihrem Aufschmelzen den PTC-Effekt unterstützen. Dies können beispielsweise hochkristalline Polyolefinwachse oder ionische Flüssigkeiten (niedrigschmelzende Metallsalze) sein. Durch die Wahl des Schmelzpunktes der Füllstoffe kann zudem die Temperatur eingestellt werden, bei der ein kaltleitendes Verhalten (PTC-Effekt) auftritt.

Erfindungsgemäß sind beide Selbstklebemassen Haftklebemassen.

Als Haftklebemassen kommen grundsätzlich alle Haftklebemassensysteme mit geeigneten haftklebenden Eigenschaften in Frage, also haftklebrige Systeme. Die zur Herstellung der Haftklebemassen dienenden Monomere werden insbesondere dermaßen gewählt, dass die resultierenden Polymere bei Raumtemperatur oder höheren Temperaturen als Haftklebemassen eingesetzt werden können.

Haftklebrig im Sinne der vorliegenden Erfindung ist eine Klebemasse, wenn sie entsprechend "Handbook of Pressure Sensitive Adhesive Technology" von Donatas Satas (van Nostrand, New York 1989) haftklebende Eigenschaften besitzt.

Zur Erzielung einer für Haftklebemassen bevorzugten Glasübergangstemperatur $T_G$ der Polymere von $T_G \leq 25\ °C$ werden die Monomere üblicherweise derart ausgesucht und die mengenmäßige Zusammensetzung der Monomermischung derart gewählt, dass diese sich in Analogie zu der von Fox vorgestellten Gleichung so verhalten (vgl. T.G. Fox, Bull. Am. Phys. Soc. 1 (1956) 123), dass sich der gewünschte Wert für die Glasübergangstemperatur $T_G$ des resultierenden Polymers ergibt nach

$$\frac{1}{T_G} = \sum_n \frac{w_n}{T_{G,n}} \qquad \text{(G1)}$$

Hierin repräsentiert n die Laufzahl über die eingesetzten Monomere, $w_n$ den Massenanteil des jeweiligen Monomers n (Gew.-%) und $T_{G,n}$ die jeweilige Glasübergangstemperatur des Homopolymers aus den jeweiligen Monomeren n in K. Als Haftklebemassen für die erste Selbstklebemasse und/oder die zweite Selbstklebemasse kommen daher beispielsweise Haftklebemassen auf der Basis von Acrylaten und/oder Methacrylaten, Naturkautschuken und/oder Synthesekautschuken in Frage.

So lassen sich also Haftklebemassen auf Basis von Acrylsäure und/oder Methacrylsäure und/oder auf Basis von Estern der vorgenannten Verbindungen einsetzen oder solche auf Basis von hydrierten Natur- oder Synthesekautschuken, da diese besonders alterungsstabil sind und somit wiederholten Heizprozessen des erfindungsgemäßen Flächenelements langzeitig standzuhalten vermögen.

Es sind insbesondere Acrylathaftklebemassen geeignet, die etwa durch radikalische Polymerisation erhältlich sind und die zumindest teilweise auf mindestens einem Acrylmonomer der allgemeinen Formel $CH_2=C(R^1)(COOR^2)$ basieren, wobei $R^1$ gleich H oder ein $CH_3$-Rest ist und $R^2$ gleich H ist oder aus der Gruppe der gesättigten, unverzweigten oder verzweigten, substituierten oder unsubstituierten $C_1$- bis $C_{30}$-Alkylreste gewählt ist. Das mindestens eine Acrylmonomer sollte einen Massenanteil von mindestens 50 Gew.-% in der Haftklebemasse aufweisen.

Es lassen sich nach einer besonders vorteilhaften Ausgestaltung ferner Polymere einsetzen, die

(a1) zumindest teilweise auf mindestens einem Acrylmonomer der allgemeinen Formel $CH_2=C(R^1)(COOR^{2'})$ basieren, wobei $R^1$ gleich H oder ein $CH_3$-Rest ist und $R^{2'}$ aus der Gruppe der gesättigten, unverzweigten oder verzweigten, substituierten oder unsubstituierten $C_2$- bis $C_{20}$-Alkylreste gewählt ist, und

(a2) zumindest teilweise auf einen mit dem mindestens einen Acrylmonomer polymerisierbaren Comonomer basieren, das insbesondere aus Vinylverbindungen mit funktionellen Gruppen, Maleinsäureanhydrid, Styrol, Styrol-Verbindungen, Vinylacetat, Acrylamiden und mit Doppelbindung funktionalisierten Photoinitiatoren gewählt werden kann.

Dabei weist vorzugsweise das mindestens eine Acrylmonomer (a1) einen Massenanteil von 65 Gew.-% bis 100 Gew.-% und das mindestens eine Comonomer (a2) einen Massenanteil von 0 Gew.-% bis 35 Gew.-% in der Selbstklebemasse auf.

Es hat sich ferner eine mittlere molekulare Masse $M_w$ (Gewichtsmittel) der Selbstklebemasse von maximal 800.000 g/mol als vorteilhaft erwiesen, insbesondere im Hinblick auf die gewünschten mechanischen Eigenschaften der Haftklebemasse.

Die mindestens eine Selbstklebemasse kann nach einer weiteren Ausführung auch Natur- oder Synthesekautschukmassen umfassen oder auf solchen basieren. Für Selbstklebemasse aus Naturkautschuk wird der Naturkautschuk bis zu einem frei wählbaren Molekulargewicht gemahlen und dann mit dem elektrisch leitfähigen Füllstoff additiviert.

Als eine besondere Ausführung hierzu können auch teilkristalline Polymere wie EVA (Ethylenvinylacetat) oder Polyolefine als Selbstklebemasse eingesetzt oder zu dieser hinzugefügt werden. Insbesondere bei einer Verwendung als erste Selbstklebemasse bieten diese Klebemassensysteme infolge der darin auftretenden Volumenvergrößerung der kristallinen Phase beim Überschreiten der Kristallitschmelztemperatur eine zusätzliche Unterstützung des PTC-Effekts.

In bevorzugter Weise werden Acryl- oder Methacrylmonomere der allgemeinen Formel $CH_2=C(R^1)(COOR^{2''})$ eingesetzt, die Acryl- und Methacrylsäureester umfassen, wobei die Gruppe $R^{2''}$ aus der Gruppe der gesättigten, unverzweigten oder verzweigten, substituierten oder unsubstituierten $C_4$- bis $C_{14}$-Alkylreste, insbesondere $C_4$- bis $C_9$-Alkylreste, gewählt ist. Spezifische Beispiele, ohne sich durch diese Aufzählung einschränken zu wollen, sind Methylacrylat, Methylmethacrylat, Ethylacrylat, n-Butylacrylat, n-Butylmethacrylat, n-Pentylacrylat, n-Hexylacrylat, n-Heptylacrylat, n-Octylacrylat, n-Octylmethacrylat, n-Nonylacrylat, Laurylacrylat, Stearylacrylat, Behenylacrylat und deren verzweigten Isomere, beispielsweise Isobutylacrylat, 2-Ethylhexylacrylat, 2-Ethylhexylmethacrylat, Isooctylacrylat oder Isooctylmethacrylat.

Weitere einsetzbare Verbindungsklassen sind monofunktionelle Acrylate bzw. Methacrylate der allgemeinen Formel $CH_2=C(R^1)(COOR^{2'''})$, wobei der Rest $R^{2'''}$ aus der Gruppe der überbrückten oder nicht überbrückten Cycloalkylreste mit mindestens 6 C-Atomen gewählt ist. Die Cycloalkylreste können auch substituiert sein, beispielsweise durch $C_1$- bis $C_6$-Alkylgruppen, Halogenatome oder Cyanogruppen. Spezifische Beispiele sind Cyclohexylmethacrylat, Isobornylacrylat, Isobornylmethacrylat und 3,5-Dimethyladamantylacrylat.

In einer bevorzugten Vorgehensweise werden Acrylmonomere und/oder Comonomere eingesetzt, die einen oder mehrere Substituenten aufweisen, insbesondere polare Substituenten, beispielsweise Carboxyl-, Sulfonsäure-, Phosphonsäure-, Hydroxyl-, Lactam-, Lacton-, N-substituierte Amid-, N-substituierte Amin-, Carbamat-, Epoxy-, Thiol-, Alkoxy-, Cyan-, Halogenid- und Ethergruppen.

Sehr vorteilhaft im Sinne des Acrylmonomers (a1) eignen sich Monomere, die aus der folgenden Gruppe ausgewählt sind: substituierte oder unsubstituierte Verbindungen umfassend Methylacrylat, Methylmethacrylat, Ethylacrylat, n-Butylacrylat, n-Butylmethacrylat, n-Pentylacrylat, n-Hexylacrylat, n-Heptylacrylat, n-Octylacrylat, n-Octylmethacrylat, n-Nonylacrylat, Laurylacrylat, Stearylacrylat, Behenylacrylat, Isobutylacrylat, 2-Ethylhexylacrylat, 2-Ethylhexylmethacrylat, Isooctylacrylat, Isooctylmethacrylat, Cyclohexylmethacrylat, Isobornylacrylat, Isobornylmethacrylat, und 3,5-Dimethyladamantylacrylat.

Geeignet sind ebenfalls moderat basische Comonomere (a2) wie einfach oder zweifach N-alkylsubstituierte Amide, insbesondere Acrylamide. Spezifische Beispiele sind hier N,N-Dimethylacrylamid, N,N-Dimethylmethacrylamid, N-tert-Butylacrylamid, N-Vinylpyrrolidon, N-Vinyllactam, Dimethylaminoethylacrylat, Dimethylaminoethylmethacrylat, Diethylaminoethylacrylat, Diethylaminoethylmethacrylat, N-Methylolacrylamid, N-Methylolmethacrylamid, N-(Butoxymethyl)methacrylamid, N-(Ethoxymethyl)acrylamid, N-Isopropylacrylamid, wobei auch diese Aufzählung nicht abschließend ist.

Weitere bevorzugte Beispiele für Comonomere (a2) sind Hydroxyethylacrylat, Hydroxyethylmethacrylat, Hydroxypropylacrylat, Hydroxypropylmethacrylat, Allylalkohol, Maleinsäureanhydrid, Itaconsäureanhydrid, Itaconsäure, Glyceridylmethacrylat, Phenoxyethylacrylat, Phenoxyethylmethacrylat, 2-Butoxyethylacrylat, 2-Butoxyethylmethacrylat, Cyanoethylacrylat, Cyanoethylmethacrylat, Glycerylmethacrylat, 6-Hydroxyhexylmethacrylat, Vinylessigsäure, Tetrahydrofurfurylacrylat, beta-Acryloyloxypropionsäure, Trichloracrylsäure, Fumarsäure, Crotonsäure, Aconitsäure, Dimethylacrylsäure, wobei diese Aufzählung nicht abschließend ist.

In einer weiteren bevorzugten Vorgehensweise werden als Comonomere (a2) Vinylverbindungen, insbesondere Vinylester, Vinylether, Vinylhalogenide, Vinylidenhalogenide, Vinylverbindungen mit aromatischen Cyclen und Heterocyclen in Alphastellung eingesetzt, wobei als nicht ausschließend Beispiele etwa Vinylacetat, Vinylformamid, Vinylpyridin, Ethylvinylether, Vinylchlorid, Vinylidenchlorid, Styrol und Acrylnitril genannt seien.

Besonders vorteilhaft kann das mindestens eine Comonomer (a2) ein Photoinitiator mit einer copolymerisierbaren Doppelbindung sein, insbesondere ausgewählt aus der Gruppe enthaltend Norrish-I- Photoinitiatoren oder Norrish-II-Photoinitiatoren, Benzoinacrylate oder acrylierte Benzophenone.

In einer weiteren bevorzugten Vorgehensweise werden zu den beschriebenen Comonomeren (a2) zusätzliche Monomere hinzugesetzt, die eine hohe statische Glasübergangstemperatur besitzen. Als derartige zusätzliche Monomere eigenen sich aromatische Vinylverbindungen wie etwa Styrol, wobei bevorzugt die aro-

matischen Kerne aus $C_4$- bis $C_{18}$-Bausteinen bestehen und auch Heteroatome enthalten können. Besonders bevorzugte Beispiele sind 4-Vinylpyridin, N-Vinylphthalimid, Methylstyrol, 3,4-Dimethoxystyrol, 4-Vinylbenzoesäure, Benzylacrylat, Benzylmethacrylat, Phenylacrylat, Phenylmethacrylat, t-Butylphenylacrylat, t-Butylphenylmethacrylat, 4-Biphenylacrylat und 4-Biphenylmethacrylat, 2-Naphthylacrylat und 2-Naphthylmethacrylat sowie Mischungen aus diesen Monomeren, wobei auch diese Aufzählung nicht abschließend ist.

Bevorzugt werden die für die erfindungsgemäßen Flächenelemente eingesetzten Selbstklebemassen zusätzlich vernetzt, wobei hohe Vernetzungsgrade angestrebt werden, die insbesondere auch den PTC-Effekt verstärken (vergleiche EP 0 311 142 A1 sowie US 4 775 778 A) und daher in besonderem Maß für die erste Selbstklebemasse geeignet sind. Eine Vernetzung beseitigt oder vermindert auch die Auswirkungen des NTC-Effekts (Negative Temperature Coefficient), der bisweilen bei Temperaturen oberhalb des Schmelzpunkts der ersten Selbstklebemasse beobachtet wird.

Nach einer bevorzugten Ausgestaltung der Erfindung weist ein Basispolymer der ersten Selbstklebemasse bevorzugt einen Vernetzungsgrad auf, der mindestens einem Gelwert von 35 % entspricht, insbesondere von mehr als 60 %. Als Gelwert wird vorliegend das Verhältnis von den Anteilen eines Basispolymers, die in einem geeigneten Lösungsmittel (beispielsweise Toluol oder Xylol) nicht löslich sind, zu der Summe aus löslichen Anteilen und nicht-löslichen Anteilen des Basispolymers verstanden.

Ein hoher Vernetzungsgrad kann etwa in einem Vernetzungsschritt mit Elektronenstrahlen erhalten werden. Typische Bestrahlungsvorrichtungen, die zum Einsatz kommen können, sind Linearkathodensysteme, Abtastsysteme (Scannersysteme) oder Segmentkathodensysteme, sofern es sich hierbei um Elektronenstrahlbeschleuniger handelt. Eine ausführliche Beschreibung des Standes der Technik und die wichtigsten Verfahrensparameter findet man bei Skelhorne, "Electron Beam Processing, in Chemistry and Technology of UV and EB Formulation for Coatings, Inks and Paints", Vol. 1, 1991, SITA, London. Typische Beschleunigungsspannungen liegen im Bereich zwischen 50 kV und 500 kV, vorzugsweise im Bereich zwischen 80 kV und 300 kV. Die angewandten Streudosen bewegen sich zwischen 5 kGy bis 150 kGy, insbesondere zwischen 20 kGy und 100 kGy. Es können auch andere Verfahren eingesetzt werden, die eine hochenergetische Bestrahlung ermöglichen.

Weiterhin kann erfindungsgemäß eine Variation der elektrischen Leitfähigkeit und somit der thermischen Erwärmung über den Vernetzungsgrad bewirkt werden. Durch Erhöhen der bei einer Vernetzungsreaktion einwirkenden Elektronenstrahldosis (und damit einhergehend einer Erhöhung des Vernetzungsgrades) lässt sich die elektrische Leitfähigkeit erhöhen, so dass bei einem konstanten Stromfluss durch die Heizschicht des Flächenelements die erzielbare Temperatur der Selbstklebemasse ansteigt. Ebenso lässt sich über den Vernetzungsgrad das kaltleitende Verhalten der ersten Selbstklebemasse steuern.

Zur Verringerung der für eine Vernetzungsreaktion erforderlichen Strahlendosis können der Selbstklebemasse zusätzlich Vernetzer und/oder Promotoren zur Vernetzung beigemischt werden, insbesondere solche, die mittels Elektronenstrahlen oder thermisch anregbar sind. Geeignete Vernetzer für die Elektronenstrahlvernetzung sind etwa bifunktionelle oder multifunktionelle Acrylate oder Methacrylate oder Triallylcyanurate sowie Triallylisocyanurate. Als thermisch aktivierbare Vernetzer werden bevorzugt bifunktionelle oder multifunktionelle Epoxide, Hydroxide, Isocyanate oder Silane beigemischt.

Eine Klebemasse kann selbstverständlich weitere Rezeptierungsbestandteile und/oder Zuschlagsstoffe umfassen wie zum Beispiel Hilfsstoffe, Pigmente, rheologische Additive, Additive zur Verbesserung der Haftung, Weichmacher (Plastifizierungsmittel), Harze, Elastomere, Alterungsschutzmittel (Antioxidantien), Lichtschutzmittel, UV-Absorber sowie sonstige Hilfs- und Zusatzstoffe, beispielsweise Trockenmittel (etwa Molekularsieb-Zeolithe oder Calciumoxid), Fließ- und Verlaufmittel, Benetzer wie Tenside oder Katalysatoren sowie wärmeleitende Füllstoffe, Wärme speichernde Füllstoffe oder Zuschlagstoffe, die durch Wärme freigesetzt werden oder deren Freisetzung durch Wärmeunterstützt wird.

Als Hilfsstoffe können alle fein gemahlenen festen Zusatzstoffe wie zum Beispiel Kreide, Magnesiumcarbonat, Zinkcarbonat, Kaolin, Bariumsulfat, Titandioxid oder Calciumoxid eingesetzt werden. Weitere Beispiele sind Talkum, Glimmer, Kieselsäure, Silikate oder Zinkoxid. Natürlich können auch Mischungen der genannten Stoffe eingesetzt werden.

Die eingesetzten Pigmente können organischer oder anorganischer Natur sein. Es kommen alle Arten organischer oder anorganischer Farbpigmente in Frage, beispielsweise Weißpigmente wie Titandioxid zur Verbesserung der Licht- und UV-Stabilität oder Metallpigmente.

Beispiele für rheologische Additive sind pyrogene Kieselsäuren, Schichtsilikate (beispielsweise Bentonite), hochmolekulare Polyamidpulver oder Pulver auf der Basis von Rhizinusölderivaten.

Additive zur Verbesserung der Haftung können zum Beispiel Stoffe aus den Gruppen der Polyamide, Epoxide oder Silane sein.

Beispiele für Weichmacher zur Verbesserung der Klebfähigkeit sind Phthalsäureester, Trimellitsäureester, Phosphorsäureester, Adipinsäureester sowie Ester anderer acyclischer Dicarbonsäuren, Fettsäureester, Hydroxycarbonsäureester, Alkylsulfonsäureester des Phenols, aliphatische, cycloaliphatische und aromatische Mineralöle, Kohlenwasserstoffe, flüssige oder halbfeste Kautschuke (zum Beispiel Nitrilkautschuke oder Polyisoprenkautschuke), flüssige oder halbfeste Polymerisate aus Buten und/oder Isobuten, Acrylsäureester, Polyvinylether, Flüssig- und Weichharze auf Basis der Rohstoffe, die auch die Basis für klebrigmachende Harze darstellen, Wollwachs und andere Wachse, Silikone sowie Polymerweichmacher wie etwa Polyester oder Polyurethane.

Zuschlagstoffe, die durch Wärme freigesetzt werden oder deren Freisetzung durch Wärme unterstützt wird, sind solche Systeme, die einen aktiven Stoff enthalten, der infolge der Einwirkung von Wärme freigesetzt oder aktiviert wird, so dass eine kontrollierte Abgabe dieses aktiven Stoffs möglich ist. Als aktiver Stoff kommt hierbei jeder Stoff in Frage, der bei der thermischen Freisetzung oder Aktivierung eine besondere Wirkung entfaltet, beispielsweise ein Farbstoff, ein medizinischer oder kosmetischer Wirkstoff oder ein Sprengzünder (Initialsprengstoff). Die Wirkung kann etwa infolge der Freisetzung des Stoffs (zum Beispiel bei einem topisch anwendbaren Wirkstoff) oder bei einer thermischen Aktivierung eintreten, etwa einer thermisch initiierten chemischen Reaktion (beispielsweise einer molekularen Umlagerung, einer Vernetzungsreaktion oder einer Zersetzung) oder einem thermisch initiierten physikalischen Vorgang (beispielsweise einer Adsorption/Desorption oder einem Phasenübergang). Beispielsweise kann es sich bei dem durch Wärme freisetzbaren Zuschlagstoff um einen in einer schmelzbaren Matrix verkapselten topisch anwendbaren medizinischen Wirkstoff handeln.

Die Rezeptierung der Klebemasse mit weiteren Bestandteilen wie zum Beispiel Hilfsstoffen und Weichmachern, ist ebenfalls Stand der Technik.

Zur Optimierung der klebtechnischen Eigenschaften können den erfindungsgemäßen Selbstklebemassen Harze beigemischt werden. Als zuzusetzende klebrigmachende Harze (die Klebkraft steigende Harze) sind ausnahmslos alle vorbekannten und in der Literatur beschriebenen Klebharze einsetzbar. Genannt seien stellvertretend die Pinenharze, Indenharze und Kolophoniumharze, deren disproportionierte, hydrierte, polymerisierte, veresterte Derivate und Salze, die aliphatischen und aromatischen Kohlenwasserstoffharze, Terpenharze und Terpenphenolharze sowie $C_5$- bis $C_9$- sowie andere Kohlenwasserstoffharze. Beliebige Kombinationen dieser und weiterer Harze können eingesetzt werden, um die Eigenschaften der resultierenden Klebemasse wunschgemäß einzustellen. Im Allgemeinen lassen sich alle mit dem entsprechenden Basispolymer kompatiblen (löslichen) Harze einsetzen, insbesondere sei verwiesen auf alle aliphatischen, aromatischen, alkylaromatischen Kohlenwasserstoffharze, Kohlenwasserstoffharze auf Basis reiner Monomere, hydrierte Kohlenwasserstoffharze, funktionelle Kohlenwasserstoffharze sowie Naturharze. In einer bevorzugten Auslegung werden Harze eingesetzt, die die elektrische Leitfähigkeit und die Erwärmbarkeit nicht vermindern, auch nicht über einen längeren Zeitraum.

Eine weitere vorteilhafte Ausgestaltung des Flächenelements kann durch Zugabe von einem Wärme speichernden Füllstoff zu zumindest einer der Schichten erreicht werden. Als Wärme speichernder Füllstoff wird vorliegend jeder Füllstoff mit einer hohen Wärmekapazität verstanden, insbesondere mit einer Wärmekapazität von mehr als 0,7 J/gK. Infolge der thermischen Pufferwirkung dieser Substanzen kann so ein gleichmäßiger Verlauf beim Aufheizen sowie eine verlängerte und gleichmäßige Wärmeabgabe nach Beendigung des aktiven Wärmeerzeugungsprozesses erzielt werden. Füllstoffe mit hoher Wärmekapazität, die vorteilhaft eingesetzt werden können, sind etwa Aluminium, Beryllium, Bor, Calcium, Eisen, Graphit, Kalium, Kupfer, Magnesium, Phosphor oder Verbindungen der vorgenannten Stoffe, insbesondere Aluminiumoxid und Aluminiumchlorid, Calciumcarbonat, Calciumchlorid, Kupfersulfat, Magnetit, Haematit, Magnesiumcarbonat und Magnesiumchlorid, Phosphorchlorid oder Phosphoroxid (wobei diese Stoffe darüber hinaus auch weitere Funktionen innerhalb des Flächenelements erfüllen können, etwa Kalium oder Phosphor bei Sprengzündern).

Vorteilhaft ist es auch, wenn zumindest eine der Selbstklebemasseschichten eine hohe Wärmeleitfähigkeit aufweist, insbesondere von mindestens 0,5 W/m•K, ganz besonders bevorzugt von mehr als 1 W/m•K. Dies kann etwa durch Zugabe von wärmeleitenden Füllstoffen erreicht werden, insbesondere von elektrisch isolierenden aber hochwärmeleitenden Füllstoffen wie etwa Bornitrid oder Aluminiumoxid, da durch letztere die elektrischen Eigenschaften nicht beeinflusst werden. Jedoch sind auch elektrisch leitfähige Füllstoffe mit hoher Wärmeleitfähigkeit einsetzbar, beispielsweise Silber, Aluminium oder Kupfer. Durch besonders wärmeleitfähige Haftklebemassen kann die zum Aufschmelzen einer Heißschmelzklebemasse erforderliche Energie besser eingebracht werden, was etwa zu verkürzten Zykluszeiten beim Aufbringen des erfindungsgemäßen Flächenelements auf das Verklebungssubstrat führt.

Erfindungsgemäß kann die Zusammensetzung der ersten Selbstklebemasse mit der Zusammensetzung der zweiten Selbstklebemasse identisch oder von dieser verschieden sein.

Zusätzlich zu der Heizschicht, der Kontaktierungsschicht und der Klebemassenschicht kann das erfindungsgemäße Flächenelement weitere Schichten aufweisen. So ist es etwa möglich, dass das Flächenelement weitere Schichten aus Klebemassen enthält, etwa, indem an der von dem durchbrochenen Kontaktierungselement abgewandten Seitenfläche der Heizschicht eine dritte Selbstklebemasse vorgesehen ist. Diese Klebemasse kann jede geeignete Haftklebemasse sein, beispielsweise eine Selbstklebemasse mit einer der zuvor beschriebenen Basisklebemassen.

In einer weiteren vorteilhaften Ausführung ist zumindest eine Schicht des erwärmbaren Flächenelements mit einem Mechanismus ausgestattet, der bei erstmaliger Erwärmung des Flächenelements zu einer Kohäsionserhöhung in der ersten Selbstklebemasse, der zweiten Selbstklebemasse und/oder gegebenenfalls in der dritten Selbstklebemasse führt. Dies kann beispielsweise über eine Erhöhung der Vernetzungsdichte durch eine thermisch initiierte Nachvernetzung erreicht werden, die insbesondere durch die (intrinsische) Erwärmung des Flächenelements selbst initiiert werden kann. Vorteilhafterweise wird ein solches Flächenelement daher derart verwendet, dass zunächst die Verklebung mit mindestens einem Verklebungssubstrat hergestellt wird und anschließend die erstmalige Erwärmung vorgenommen wird, während derer sich eine Verfestigung der Verklebung einstellt.

Üblicherweise ist das Flächenelement trägerfrei ausgebildet, da hierdurch eine maximale Flexibilität des Flächenelements insgesamt gewährleistet ist. Darüber hinaus kann in dem Flächenelement aber auch ein flexibler permanenter Träger vorgesehen sein. Dieser Träger kann etwa eingesetzt werden, um die mechanischen Eigenschaften wie beispielsweise die Durchstoßfestigkeit des Flächenelements insgesamt zu verbessern. Als derartige permanente Träger können alle geeigneten Trägermaterialien verwendet werden, etwa Folien aus Metall und/oder Kunststoffen, textile Flächenelemente (beispielsweise Gewebe, Gelege, Gewirke, Vliese) oder Kombinationen aus solchen Materialien. Auch diese permanenten Träger können vollflächig geschlossen oder durchbrochen ausgebildet sein. Ist ein derartiger permanenter Träger vorgesehen, so ist es erfindungsgemäß allerdings zwingend, dass dieser nicht in direktem Kontakt mit dem Kontaktierungselement steht, sondern allenfalls an einer der Selbstklebemassenschichten angeordnet ist.

Vorteilhaft ist es dabei, wenn auch der permanente Träger zusätzlich zu seiner hohen Flexibilität eine hohe Wärmeleittähigkeit aufweist, insbesondere eine Wärmeleitfähigkeit von mindestens 0,5 W/m•K oder sogar von mehr als 1 W/m•K. Besonders bevorzugte Materialien sind mit wärmeleitfähigen Füllstoffen wie etwa Bornitrid oder Aluminiumoxid gefüllte Polymere. Derartige permanente Träger weisen typischerweise eine Stärke von weniger als 50 $\mu$m auf, bevorzugt von weniger als 25 $\mu$m, um die Flexibilität des Aufbaus insgesamt nicht zu beeinträchtigen.

In einer besonders vorteilhaften Ausführung ist der permanente Träger als polymerer Schaum ausgebildet, da hierdurch die Flexibilität des gesamten Flächenelements nicht wesentlich beeinträchtigt wird.

Ferner kann das Flächenelement an seiner ersten selbstklebenden Seitenfläche und/oder an seiner zweiten selbstklebenden Seitenfläche einen temporären Träger aufweisen. Als derartiger temporärer Träger kann jedes trennende Abdeckmaterial verwendet werden, etwa ein Trennpapier oder ein Prozessliner, der eine der äußeren Selbstklebemassen zumindest teilweise bedeckt. Als Abdeckmaterial eignen sich beispielsweise alle silikonisierten oder fluorierten Folien mit einer Releasewirkung, die rückstandsfrei wiederablösbar sind. Als Folienmaterialien seien hier nur beispielhaft PP (Polypropylen), BOPP (biaxial orientiertes Polypropylen), MOPP (monoaxial orientiertes Polypropylen), PET (Polyethylenterephthalat), PVC (Polyvinylchlorid), PUR (Polyurethan), PE (Polyethylen), PE/EVA (Polyethylen-Ethylenvinylacetat-Copolymere) und EPDM (Ethen-Propylen-Dien-Terpolymere) genannt. Weiterhin lassen sich auch Trennpapiere einsetzen, zum Beispiel Glassine-Papiere, Kraft-Papiere oder polyolefinisch beschichtete Papiere.

Besonders vorteilhaft werden Abdeckmaterialien eingesetzt, die selbst eine hohe Wärmeleitfähigkeit aufweisen, insbesondere von mindestens 0,5 W/m•K oder sogar von mehr als 1 W/m•K. Besonders bevorzugte Materialien sind mit wärmeleitfähigen Füllstoffen, wie etwa Bornitrid oder Aluminiumoxid gefüllte Polymere.

Das Flächenelement umfasst also mindestens eine Schicht, innerhalb derer Wärme erzeugt werden kann, wobei diese Schicht haftklebrig ist, zumindest eine weitere Schicht, die haftklebrig ist, sowie - zwischen diesen Schichten - eine diskontinuierliche elektrisch leitfähige Schicht, die zumindest eine Elektrode (einen Pol) der Kontaktierungsschicht darstellt. Wichtig ist hierbei, dass das Kontaktierungselement nicht auf eine Trägerschicht aufgetragen ist, sondern vielmehr unmittelbar zwischen der Heizschicht und der Klebemassenschicht angeordnet ist.

Zur Herstellung der erfindungsgemäßen Flächenelemente sind ohne Ausnahme alle bekannten und geeigneten Verfahren einsetzbar. So lassen sich die polymeren Haftklebeschichten oder Heißschmelzklebeschichten des erfindungsgemäßen Flächenelements mit den geläufigen Verfahren zur Herstellung polymerer Flächenelemente nach dem Stand der Technik herstellen. Dazu zählen etwa die Flachfolienextrusion, die Blasfolienextrusion, das Kalanderverfahren, die Beschichtung aus einer Lösung, aus einer Dispersion oder aus einer monomeren oder einer präpolymeren Vorstufe des Polymeren.

Zur Herstellung der Flächenelemente wird üblicherweise eine der beiden Selbstklebemassen zunächst schichtförmig ausgebreitet, etwa auf einem permanenten Träger oder auf einem Fertigungsträger - einem so genannten Prozessliner -, der während des Verfahrens oder spätestens am Ende des Verfahrens von dem Flächenelement wieder getrennt wird. Auf diese Selbstklebemassenschicht wird das Kontaktierungselement aufgebracht. Nach dem Aufbringen des Kontaktierungselements wird die andere Selbstklebemasse auf die frei liegende Seitenfläche des Kontaktierungselements aufgebracht.

Natürlich ist es auch möglich, das erfindungsgemäße Flächenelement in einem hiervon abweichenden beliebigen anderen Herstellungsverfahren zu erhalten, beispielsweise, indem das Kontaktierungselement zuerst auf einen Prozessliner aufgetragen wird, anschließend mit der einen Selbstklebemasse verbunden wird, der Prozessliner von dem Kontaktierungselement abgezogen wird und die andere Selbstklebemasse auf die nunmehr frei liegende Seitenfläche des Kontaktierungselements aufgebracht wird.

Zum Auftragen des Kontaktierungselements auf die eine Selbstklebemasse oder gegebenenfalls den Prozessliner können alle bekannten Verfahren eingesetzt werden, etwa das Aufbringen (beispielsweise Drucken) von Leitlacken, Leitpasten oder Leittinten, der Transfer von Blechen, Folien oder Schichten (beispielsweise solchen aus Metallen) mittels Heißprägen, Heißsiegeln, Aufkaschieren oder Auflaminieren oder das diskontinuierliche Aufbringen von Mischungen aus Polymeren und leitfähigen Füllstoffen (beispielsweise Polymer-Ruß-Compounds), wobei in letzterem Fall das Kontaktierungselement eine Leitfähigkeit aufweisen muss, die mindestens um den Faktor 10 höher ist als die Leitfähigkeit der intrinsisch erwärmbaren ersten Selbstklebemasse.

In einer einfachen Ausgestaltung eines solchen Verfahrens wird die erwärmbare erste Selbstklebemasse mit einem elektrisch leitfähigen Metallgitter kontaktiert. In bevorzugter Weise werden Metalle eingesetzt, die über einen längeren Zeitraum nicht oder nur wenig korrodieren. In sehr bevorzugten Ausführungen wird beispielsweise Kupfer oder Aluminium eingesetzt, wobei aber auch Silber- oder Gold-Kontaktierungen vorgenommen werden können.

In einer bevorzugten Ausführungsform kann das Metall direkt auf der Selbstklebemasse abgeschieden werden, etwa durch galvanische oder Bedampfungsverfahren oder durch Laserverfahren, es kann aber auch in Form einer kontinuierlichen oder durchbrochenen Schicht aufkaschiert werden, indem es von einem Prozessliner transferiert wird.

Bei der Verwendung von Leitlack, einer leitfähigen Tinte, einer leitfähigen Druckfarbe, einem intrinsisch leitfähigen Polymer oder einer Polymer-Leitstoff-Mischung werden Druckverfahren bevorzugt, insbesondere etwa ein Siebdruck, da die diskontinuierlichen Kontaktierungsschichten hiermit besonders leicht, variabel und reproduzierbar aufgebracht werden können. Der Druck kann dabei aus einer Lösung, aus einer Dispersion oder aus einer Schmelze erfolgen.

Bei der Herstellung des erfindungsgemäßen Flächenelements kann es insbesondere vorteilhaft sein, dieses nach einem Verfahren durchzuführen, bei dem zunächst eine erste Klebeschichtung aus einer der beiden Selbstklebemassen erzeugt wird (etwa, indem die Klebemasse auf einen Prozessliner aufgetragen wird) und bei dem dann auf die Oberseite der so erhaltenen Schichtung das durchbrochene Kontaktierungselement direkt aufgebracht wird, wobei dies gegebenenfalls unter Andruck erfolgen kann, und bei dem schließlich die zweite Klebeschichtung aus der anderen Selbstklebemasse auf die Oberfläche des durchbrochenen Kontaktierungselements aufgetragen wird.

Die Flächenelemente werden erfindungsgemäß zum Verbinden zweier Verklebungssubstrate miteinander oder auch zum Verbinden zweier unterschiedlicher Teilbereiche eines einzigen Verklebungssubstrats eingesetzt. Da das Flächenelement doppelseitig selbstklebend ausgebildet ist, ist es dafür angepasst, die Oberflächen zweier Verklebungssubstrate miteinander klebend zu verbinden. Insbesondere findet das Flächenelement zur Verklebung von Verklebungssubstraten in der Fahrzeugindustrie Verwendung und wird etwa in Automobilen, Bussen, Eisenbahnen, Schiffen oder Flugzeugen eingesetzt.

Das erfindungsgemäße Flächenelement kann hierbei als Bestandteil eines Verklebungsverbunds vorliegen. Ein Verklebungsverbund ist vorliegend jeglicher mittels Verklebung erhaltener Verbund aus einem Flächenelement und mindestens einem Verklebungssubstrat, das entweder mit der ersten selbstklebenden Seitenfläche oder mit der zweiten selbstklebenden Seitenfläche des Flächenelements direkt oder über weitere Bestandteile verklebt ist. Voreilhafterweise wird als Verklebungssubstrat eine Spiegelscheibe eingesetzt, insbesondere die Rückseite der verspiegelten Seite einer Spiegelscheibe, oder - im Falle eines transparenten Flächenelements - eine Sichtscheibe, beispielsweise ein Displayfenster oder eine Windschutzscheibe. Demzufolge wird das erfindungsgemäße Flächenelement zum Beheizen eines derartigen Verklebungsverbunds eingesetzt.

So kann das erfindungsgemäße Flächenelement etwa als Spiegelheizung (Außen- und Innenspiegel), in einer heizbaren Innenverkleidung (Befestigung, Geräuschdämmung, Heizung), zur Wischwasserbeheizung oder Einfriersicherung, für eine Tankbeheizung (insbesondere für Dieselfahrzeuge), für die Beheizung von Kraftstoffleitungen (gleichzeitig als Befestigung), in einer Heizung für Enteisungssysteme (Tragflächenenteisung, gegebenenfalls einschließlich Befestigungsfunktionen), in einer Lenkradheizung, zur Heizungslufterwärmung (Zusatzheizung bei kaltem Motor) oder zur Ansaugluftvorwärmung (Verbrennungsluft) eingesetzt werden. Diese Aufzählung ist rein exemplarisch und die Anwendung des erfindungsgemäßen Flächenelements nicht allein auf diese konkreten Beispiele beschränkt.

Darüber hinaus lassen sich eine Vielzahl weiterer Anwendungen finden, zum Beispiel (ohne sich durch diese Auswahl einzuschränken) zum Vermeiden von Kondenswasser oder von Niederschlag auf Flächen (beispielsweise bei Badezimmerspiegeln, zur Befestigung und Heizung, als Antibeschlag-Kaschierung etwa für Badezimmeranwendungen, als beheizbare Fliesenklebefolie, an Korrektur- oder Sonnenbrillen oder in Brillenetuis), als Sitzheizung (beispielsweise in Automobilen, einschließlich einer integrierten Anwendung von Sitzheizung und Sitzbelegungssensor für Airbags), für Sitzgelegenheiten an Bushaltestellen, in Sportstadien, bei der Gastronomie im Außenbereich oder für Toilettensitze, in Heizdecken oder Heizmatten, in Warmhalteplatten (etwa solche für Lebensmittel und Speisen, aber auch in Hochgebirgskochern oder Hochgebirgsherden, insbesondere unter Verwendung von Solarzellen), in Schuhwärmern (etwa als Einlegesohle), in Bandheizungen (etwa für Rohrleitungen, Kessel und dergleichen), zur Raumbeheizung (beispielsweise in Wandheizungen, Fußbodenheizungen oder auch als faltbare Zeltheizung), in Wasserbettheizungen, in heizbaren Gehäusen (beispielsweise als so genannte Thermobox zur Temperierung des Gehäuseinhalts oder im Elektronikbereich, etwa um im Zusammenspiel mit einem Peltier-Element in HiFi-Anlagen eine konstante Temperatur zur gewährleisten), für Motorräder (beispielsweise als Lenkerheizung oder Sattelheizung), als Gewächshausheizung (beispielsweise als großflächige Strahlungsheizung oder Konvektionsheizung oder als kleinflächige lokale Heizung direkt an den Pflanzen, etwa als Wurzelheizung), für funktionell beheizbare Bekleidung (beispielsweise in Motorradfahrerbekleidung, Autofahrerbekleidung oder Winterbekleidung), zum Beheizen und gegebenenfalls Befestigen von Anzeigesystemen (beispielsweise von LCDs, OLEDs und elektrophoretischen Displays, etwa als Einfrierschutz für Displays in Kameras oder Außenbereichsanzeigen, oder in Kirchturmuhren, etwa zum Enteisen derselben), zum Beheizen von beheizten Außenschaltern, zur Dacherwärmung (beispielsweise als Abtauanlage für Dachflächen oder Dachrinnen), in Brutkästen (beispielsweise für Jungtiere, zum Ausbrüten von Eiern oder in Inkubatoren für menschliche Babies), in der medizinischen Therapie (beispielsweise in der Wärmetherapie, als Wärmepflaster sowie für transdermale therapeutische Systeme und für transdermale Medikamentierung, so genanntes transdermal drug delivery) oder als Sprengzünder.

Entsprechend der jeweils eingesetzten Selbstklebemasse wird dabei das Flächenelement lediglich unter Andruck an dem Verklebungssubstrat fixiert

Weitere Vorteile und Anwendungsmöglichkeiten gehen aus den Ausführungsbeispielen hervor, die anhand der beigefügten Zeichnungen im folgenden näher beschrieben werden sollen. Dabei zeigt

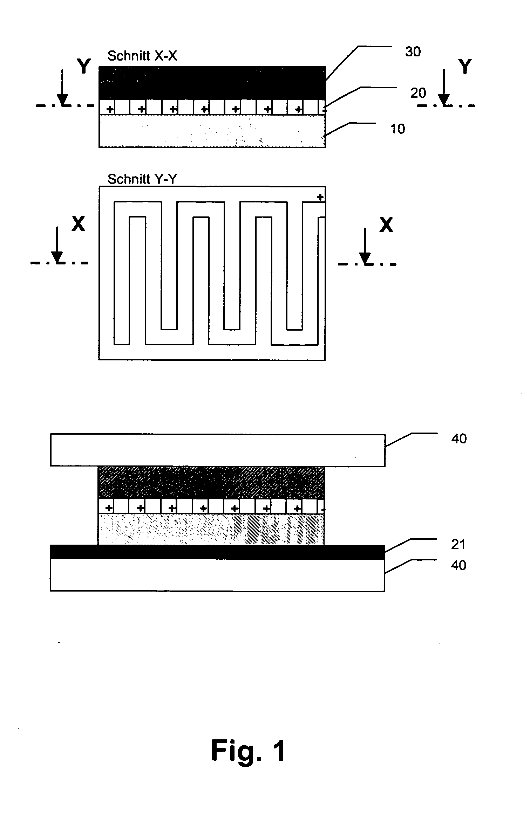

Fig. 1 im oberen Teil eine schematische Darstellung eines Längsschnitts durch ein erfindungsgemäßes Flächenelement mit einem durchbrochenen Kontaktierungselement mit Kammstruktur, bei dem alle Teilbereiche miteinander durch das durchbrochene Kontaktierungselement elektrisch leitend verbunden sind, im mittleren Teil eine schematische Darstellung eines Horizontalschnitts über das obige Flächenelement und im unteren Teil eine schematische Darstellung eines Längsschnitts durch das erfindungsgemäße Flächenelement im verklebten Zustand an einem oberen Substrat und einem unteren Substrat mit einer Gegenelektrode;

Fig. 2 im oberen Teil eine schematische Darstellung eines Längsschnitts durch ein erfindungsgemäßes Flächenelement mit einem durchbrochenen Kontaktierungselement mit doppelter Kammstruktur, bei der zwei Teilbereiche nicht über das durchbrochene Kontaktierungselement elektrisch leitend verbunden sind, und im unteren Teil eine schematische Darstellung eines Horizontalschnitts durch das obige Flächenelement;

Fig. 3 eine schematische Darstellung eines Längsschnitts durch ein erfindungsgemäßes Flächenelement mit einem durchbrochenen Kontaktierungselement mit doppelter Kammstruktur und mit einem permanenten Träger als Verklebungssubstrat;

Fig. 4 eine schematische Darstellung eines Längsschnitts durch ein erfindungsgemäßes Flächenelement mit einem durchbrochenen Kontaktierungselement mit doppelter Kammstruktur und einer dritten Selbstklebemasse;

Fig. 5 eine schematische Darstellung eines Längsschnitts durch ein erfindungsgemäßes Flächenelement, das mit einem temporären Träger abgedeckt ist;

Fig. 6 eine Messdatenkurve, in der der für unterschiedliche Temperaturen bestimmte Ohmsche Widerstand eines erfindungsgemäßen Flächenelements (Beispiel 1) grafisch dargestellt ist;

Fig. 7 eine Messdatenkurve, in der der für unterschiedliche Temperaturen bestimmte Ohmsche Widerstand eines weiteren erfindungsgemäßen Flächenelements (Beispiel 2) grafisch dargestellt ist;

Fig. 8 eine Messdatenkurve, in der der für unterschiedliche Temperaturen bestimmte Ohmsche Widerstand eines kommerziellen Flächenelements als Referenzbeispiel (Vergleichsbeispiel 1) grafisch dargestellt ist;

Fig. 9 eine schematische Darstellung eines Längsschnitts durch Prüfaufbauten zur Bestimmung der Flexibilität (Kälteschlagfestigkeit) und Spaltmaßüberbrückung; und

Fig. 10 ein Balkendiagramm mit Messdaten aus den Untersuchungen zur Bestimmung der Kälteschlagfestigkeit für unterschiedliche Flächenelemente.

Jedes der in Folgenden exemplarisch beschriebenen Flächenelemente weist eine erwärmbare erste Selbstklebemasse 10, ein Kontaktierungselement 20 und eine zweite Selbstklebemasse 30 auf.

In Fig. 1 ist ein erfindungsgemäßes Flächenelement mit einer ersten Selbstklebemasse 10, einem Kontaktierungselement 20 und einer zweiten Selbstklebemasse 30 dargestellt. Das Flächenelement weist dabei keine stabilisierende Trägerfolie auf, die die Flexibilität herabsetzt. Die erste Selbstklebemasse 10 und die zweite Selbstklebemasse 30 sind jeweils haftklebrig. Innerhalb der ersten Selbstklebemasse 10 als Heizschicht wird bei einem Stromfluss Wärme erzeugt. Das Kontaktierungselement 20 dient als zwischen den Klebeschichtungen angeordnete diskontinuierliche elektrisch leitfähige Schicht der Kontaktierung der ersten Selbstklebemasse 10.

Das Kontaktierungselement 20 weist hier eine Kammstruktur gleichmäßigen Querschnitts auf, bei dem die Finger im oberen Teilbereich auf derselben Seite des Hauptstrangs abzweigen wie die Finger im unteren Teilbereich. Wie aus dem mittleren Teil von Fig. 1 erkennbar ist, sind alle Teilbereiche des Kontaktierungselements 20 durchgängig miteinander verbunden, so dass dieses in der Schicht als eine einzige Kontaktelektrode (Pol) der intrinsisch erwärmbaren ersten Selbstklebemasse dienen kann (dargestellt durch das - willkürlich gewählte - Symbol "+"). Im Verbund mit den Verklebungssubstraten 40 ist daher eine weitere Kontaktelektrode als externe Gegenelektrode 21 erforderlich, damit ein Strom durch die erste Selbstklebeschicht fließen kann. Diese externe Gegenelektrode ist vorliegend auf der Oberseite des unteren Verklebungssubstrats 40 als dünne metallische Schicht aufgebracht. Im Zusammenwirken von Kontaktierungselement 20 und externer Gegenelektrode 21 ist ein Stromfluss durch die erste Selbstklebemasse möglich, der im wesentlichen senkrecht zur Flächenausdehnung der ersten Selbstklebemasse verläuft (also in z-Richtung).

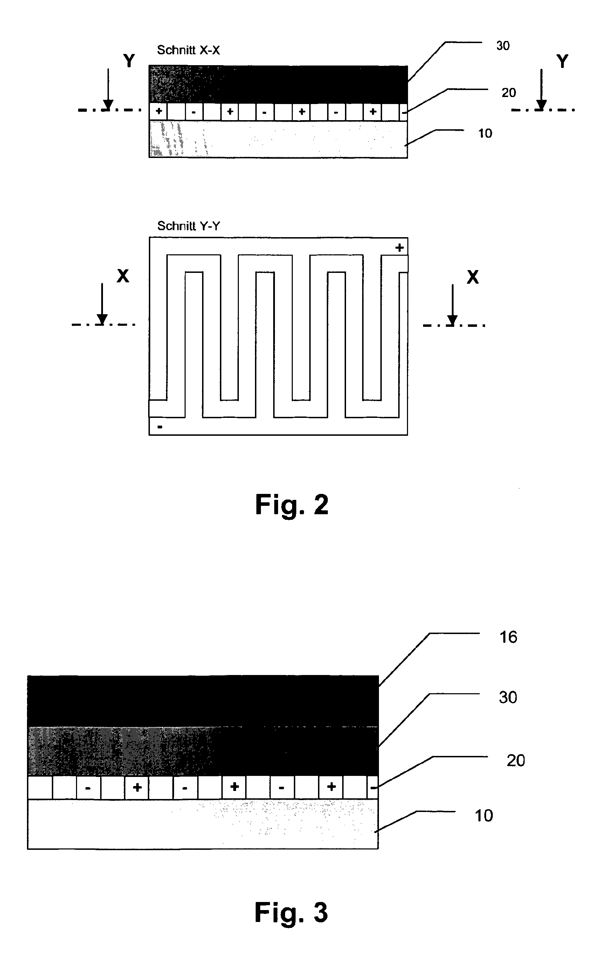

In Fig. 2 ist ein weiteres erfindungsgemäßes Flächenelement mit einer ersten Selbstklebemasse 10, einem Kontaktierungselement 20 und einer zweiten Selbstklebemasse 30 dargestellt. Auch hier sind die erste Selbstklebemasse 10 und die zweite Selbstklebemasse 30 jeweils entweder haftklebrig oder heißschmelzklebrig. Innerhalb der ersten Selbstklebemasse 10 als Heizschicht wird bei einem Stromfluss Wärme erzeugt. Das Kontaktierungselement 20 dient als zwischen den Klebeschichtungen angeordnete diskontinuierliche elektrisch leitfähige Schicht der Kontaktierung der ersten Selbstklebemasse 10.

Das Kontaktierungselement 20 weist auch hier eine Kammstruktur gleichmäßigen Querschnitts auf. Wie aus dem unteren Teil von Fig. 2 erkennbar ist, sind der obere Teilbereich des Kontaktierungselements 20 und der untere Teilbereich des Kontaktierungselements 20 jedoch nicht durchgängig miteinander verbunden, so dass jeder der beiden Teilbereiche als jeweils eine Kontaktelektrode der intrinsisch erwärmbaren ersten Selbstklebemasse dienen kann und das Kontaktierungselement daher beide Kontaktelektroden enthält (dargestellt durch die - willkürlich gewählten - unterschiedlichen Symbole "+" und "-"), weswegen eine externe Gegenelektrode nicht erforderlich ist. Im Zusammenwirken der beiden Teilbereiche des Kontaktierungselements 20 ergibt sich ein Stromfluss durch die erste Selbstklebemasse, der im wesentlichen innerhalb der Ebene der Flächenausdehnung der ersten Selbstklebemasse verläuft (also in der xy-Ebene) und nur um ein Weniges senkrecht zu dieser.

Das in Fig. 3 dargestellte Flächenelement ist mit dem in Fig. 2 dargestellten Flächenelement im Hinblick auf die Ausgestaltung und Anordnung der ersten Selbstklebemasse 10, des Kontaktierungselements 20 und der zweiten Selbstklebemasse 30 identisch. Im Unterschied zu dem in Fig. 2 dargestellten Aufbau weist das in Fig. 3 dargestellte Flächenelement jedoch einen permanenten Träger 16 auf, der als (oberes) Verklebungssubstrat auf der zweiten Selbstklebemasse 30 angeordnet ist.

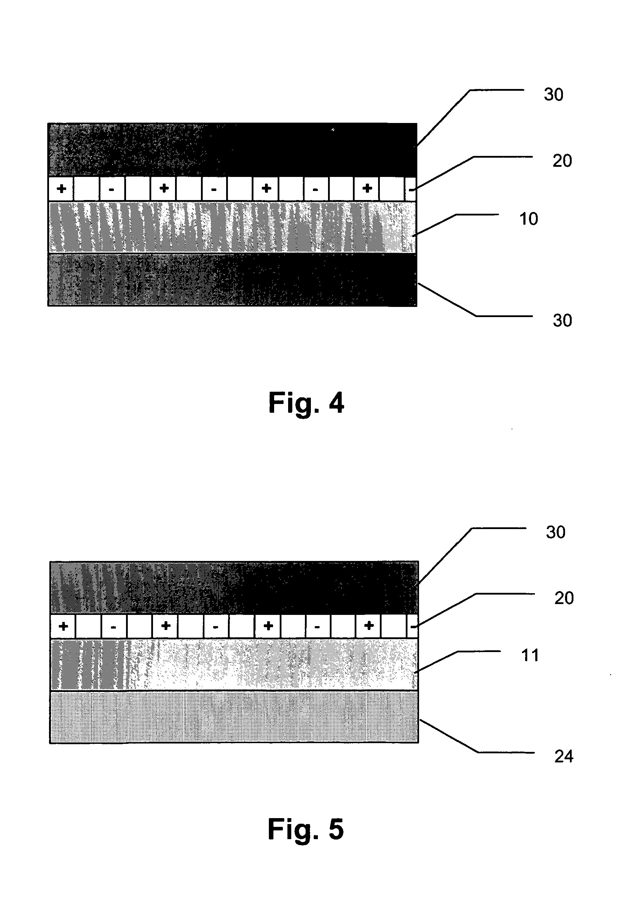

Das in Fig. 4 dargestellte erfindungsgemäße Flächenelement ist mit dem in Fig. 2 dargestellten Flächenelement im Hinblick auf die Ausgestaltung und Anordnung der ersten Selbstklebemasse 10, des Kontaktierungselements 20 und der zweiten Selbstklebemasse 30 identisch. Im Unterschied zu dem in Fig. 2 dargestellten Flächenelement weist das in Fig. 4 dargestellte Flächenelement jedoch an der unteren Seite der ersten Selbstklebemasse 10 eine dritte Selbstklebemasse 30 auf, die ein besseres Verkleben des Flächenelements auf einem Verklebungssubstrat ermöglicht. Da vorliegend das Kontaktierungselement 20 beide Kontaktierungselektroden der ersten Selbstklebemasse enthält, kann die dritte Selbstklebemasse beliebig gewählt werden, etwa - wie in dem in Fig. 4 gezeigten Beispiel - identisch zu der zweiten Selbstklebemasse 30.

Das in Fig. 5 dargestellte erfindungsgemäße Flächenelement ist mit dem in Fig. 2 dargestellten Flächenelement im Hinblick auf die Ausgestaltung und Anordnung des Kontaktierungselements 20 und der zweiten Selbstklebemasse 30 identisch, eine Abweichung besteht in der als intrinsisch erwärmbare kaltleitende Haftklebemasse 11 ausgebildeten ersten Selbstklebemasse. Zum Schutz der Haftklebemasse 11 vor einem unbeabsichtigten Verkleben bereits bei zufälliger Berührung ist die Haftklebemasse 11 an der Außenseite des Flächenelements unten mit einem temporären Träger 24 zumindest teilweise abgedeckt.

Die Erfindung wird im Folgenden durch einzelne exemplarisch ausgewählte Experimente beschrieben, ohne sich durch die Wahl der untersuchten Proben unnötig beschränken zu wollen.

Es wurden die nachfolgend aufgeführten Testmethoden zur Charakterisierung der erfindungsgemäßen Flächenelemente angewendet:

Die Bestimmung der Klebkraft intrinsisch erwärmbarer Haftklebemassen (Test A) erfolgte in einem Schälversuch auf einer Stahlplatte unter einem Winkel von 180 ° bei einer Abzugsgeschwindigkeit von 300 mm/min entsprechend ASTM D 3330-04. Alle Messungen wurden bei Raumtemperatur (23 °C) unter klimatisierten Bedingungen (bei 50 % relativer Luftfeuchtigkeit) durchgeführt.

Die Bestimmung der Klebkraft intrinsisch erwärmbarer Heißschmelzklebemassen (Test B) erfolgte in einem T-Schälkrafttest. Hierzu wurde ein 200 $\mu$m dicker Streifen der zu untersuchenden Heißschmelzklebemasse unter Vakuum auf eine unbehandelte Polyesterfolie (Mitsubishi H) mittels einer Heizpresse bei einer Temperatur von 140 °C gesiegelt. Aus dem so erhaltenen Verbundsystem wurde ein 20 mm breiter Streifen geschnitten und dieser für 24 h im Raumklima konditioniert. Anschließend wurde die Heizfolie bei Raumtemperatur unter klimatisierten Bedingungen wieder vom Polyesterträger abgezogen und die dafür benötigte Kraft gemessen. Dabei wurden weder die Heißschmelzklebemasse noch die Polyesterfolie gestützt oder fixiert, so dass sich ein T-förmiges Abschälen ergab. Die Messergebnisse sind in N/cm angegeben und aus drei Messungen gemittelt.

Die Bestimmung der elektrischen Erwärmbarkeit (Test C) erfolgte für ein Flächenelement, indem die Temperaturerhöhung nach Anlegen einer elektrischen Spannung gemessen wurde. Die Messung der Temperatur erfolgte mit einem Pt100-Thermofühler. Das erfindungsgemäße Flächenelement und das Vergleichsbeispiel wurde mit der haftklebrigen Seite auf eine Glasplatte appliziert. An das flexible Heizelement wurde mittels eines Transformators eine Gleichspannung von 12,8 Volt angelegt. Die Temperatur wurde nach einer Zeit von 600 s direkt auf der Oberfläche der Glasplatte gemessen. Die Messergebnisse sind in °C angegeben.

Im Rahmen desselben Tests erfolgte die Ermittlung des Ausmaßes des PTC-Effekts bei den gleichen Testmustern; hierfür wurde der zeitliche Verlauf der sich einstellenden Temperatur nach Strombeaufschlagung aufgezeichnet. Die Messung der Temperatur erfolgte dabei wie vorstehend beschrieben. Weiterhin wurden Strom und Spannung im zeitlichen Verlauf aufgezeichnet, so dass nach dem Ohmschen Gesetz daraus die Widerstandsänderung berechnet werden konnte.

Als Beispiele für erfindungsgemäße Flächenelemente wurden Flächenelemente mit einer Haftklebemasse als ersten Selbstklebemasse hergestellt.

Für die intrinsisch erwärmbare Haftklebemasse wurde zunächst eine Basishaftklebemasse analog der Offenbarung in EP 04 712 016 hergestellt, die eine Comonomerzusammensetzung von 44,5 Gew.-% 2-Ethylhexylacrylat, 44,5 Gew.-% n-Butylacrylat, 8 Gew.-% Methylacrylat und 3 Gew.-% Acrylsäure besaß. Die Bestimmung des Molekulargewichts ergab ein mittleres Molekulargewicht $M_w$ von 650000 g/mol bei einer Polydispersität $M_w/M_n$ von 7,0. Die so erhaltene Basishaftklebemasse wurde in Lösung mit 40 Gew.-% Graphit (Timcal Timrex KS 6) abgemischt und anschließend mittels eines Streichbalkens auf ein silikonisiertes Glassine-Trennpapier (Fa. Laufenberg) aufgetragen. Nach Trocknen für 10 Minuten bei 120 °C betrug die Dicke der so erhaltenen Haftklebemassenschicht 100 $\mu$m.

Anschließend wurde diese Haftklebemasse durch Elektronenbestrahlung vernetzt. Die Elektronenbestrahlung erfolgte mit einem Gerät der Fa. Electron Crosslinking AB, Halmstad, Schweden. Das beschichtete Haftklebeband wurde dabei über eine standardmäßig vorgesehene Kühlwalze unter dem Lenard-Fenster des Beschleunigers hindurch geführt. Dabei wurde in der Bestrahlungszone der Luftsauerstoff durch Spülen mit reinem Stickstoff verdrängt. Die Bahngeschwindigkeit betrug 10 m/min. Die Elektronenstrahldosis betrug hierbei 50 kGy bei einer Beschleunigungsspannung von 180 kV für Beispiel 1.

Für die intrinsisch erwärmbare Heißschmelzklebemasse (Vergleichsversuche) wurde als Basisheißschmelzklebemasse ein Ethylen-Vinylacetat-Copolymer (EVA) des Typs Escorene Ultra FL 00728 (ExxonMobil) mit 28 Gew.-% Vinylacetatgehalt verwendet. In diese Basisheißschmelzklebemasse wurde mittels eines Meßkneters vom Typ Haake Rheomix bei einer Temperatur von 140 °C und einer Drehzahl von 120 min$^{-1}$ 14 Gew.-% Leitruß (Printex XE2; Degussa) über einen Zeitraum von 45 min eincompoundiert. Aus dem so erhaltenen Polymercompound wurde mittels einer Vakuumpresse ein Flächenelement mit einer Dicke von 200 $\mu$m hergestellt.

Erfindungsgemäßes Beispiel

Für Beispiel 1 wurde der in Fig. 2 dargestellte Aufbau unter Verwendung der zuvor beschriebenen intrinsisch erwärmbaren Haftklebemasse mit einer Dicke von 100 $\mu$m als erste Selbstklebemasse, der zuvor beschriebenen Basishaftklebemasse mit einer Dicke von 75 $\mu$m als zweite Selbstklebemasse und einem kammförmig aus Kupferfolie von 0,03 mm Stärke ausgeschnittenen zweiteiligen Kontaktierungselement eingesetzt, das einen Abstand von 1,5 mm besaß. Die erwärmbare Fläche hatte eine Größe von 180 cm$^2$.

Vergleichsbeispiele

Für Vergleichsbeispiel A wurde der in Fig. 2 dargestellte Aufbau unter Verwendung der zuvor beschriebenen intrinsisch erwärmbaren Heißschmelzklebemasse mit einer Dicke von 150 $\mu$m als erste Selbstklebemasse, der zuvor beschriebenen Basishaftklebemasse mit einer Dicke von 75 $\mu$m als zweite Selbstklebemasse und einem kammförmig aus Kupferfolie von 0,03 mm Stärke ausgeschnittenen zweiteiligen Kontaktierungselement eingesetzt, das einen Abstand von 1,5 mm besaß. Die Leiterbahnen des Kontaktierungselements wurden bei einer Temperatur von 140 °C auf die Heißschmelzklebemasse gesiegelt. Die erwärmbare Fläche hatte eine Größe von 180 cm$^2$.

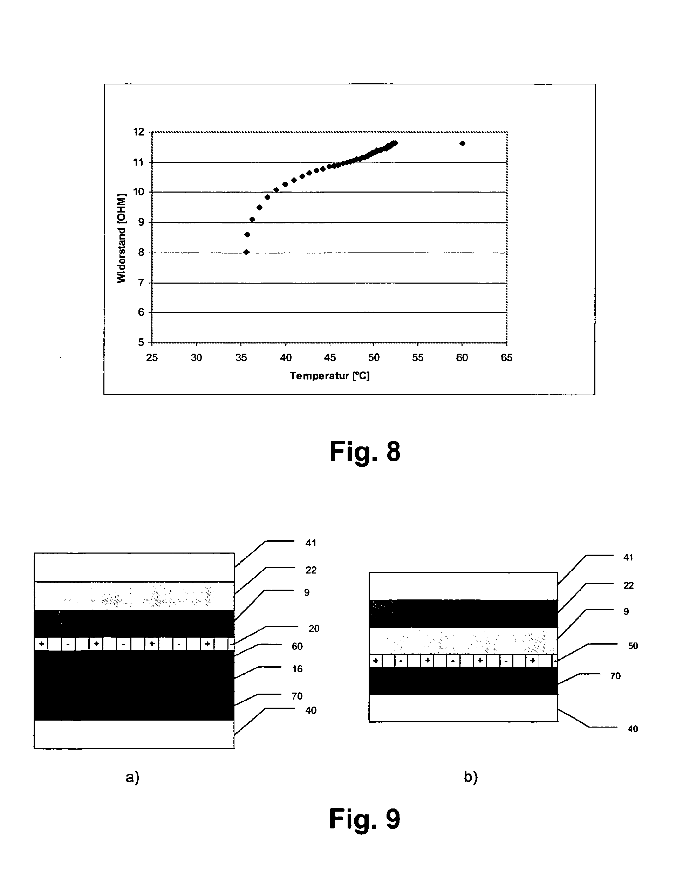

Für Vergleichsbeispiel B wurde der in Fig. 9 b) dargestellte Aufbau (ohne Substrat 40 und Glasplatte 41) mit der zuvor beschriebenen intrinsisch erwärmbaren Heißschmelzklebemasse 9 in einer Dicke von 150 $\mu$m sowie mit der zuvor beschriebenen Basishaftklebemasse 22 in einer Dicke von 75 $\mu$m hergestellt. Die Leiterbahnen wurden mit einem Silberleitlack hergestellt, der direkt auf die erwärmbare Heißschmelzklebemasse aufgetragen wurde. Dieser Aufbau ist in seiner Funktionalität hinsichtlich Kleben und Heizen direkt vergleichbar mit den Vergleichsbeispielen.

Für Vergleichsbeispiel 1 wurde ein kommerziell verfügbares PTC-Heizelement nach dem Stand der Technik von einem Außenspiegel der Firma Porsche verwendet.

Für Vergleichsbeispiel 2 wurde der in Fig. 9 a) dargestellte Aufbau (ohne Substrat 40 und Glasplatte 41) mit der zuvor beschriebenen intrinsisch erwärmbaren Heißschmelzklebemasse 9 in einer Dicke von 150 μm sowie mit der zuvor beschriebenen Basishaftklebemasse 22 in einer Dicke von 75 μm hergestellt. Dieser Aufbau unterscheidet sich von Vergleichsbeispiel B lediglich in der Verwendung einer flexiblen Leiterplatte aus 30 μm dicken Kupferleitbahnen auf einer 75 μm dicken Polyesterfolie zur Kontaktierung der erwärmbaren Heißschmelzklebemasse, so dass ein Vergleich der Eigenschaften dieser beiden Muster die Vorteile einer trägerfreien Ausbildung des Flächenelements als besondere Ausführungsform der Erfindung gegenüber einer Ausbildung des Flächenelements mit einem permanenten Träger unmittelbar vor Augen führen kann.

Die Klebkraft wurde für die zuvor beschriebene Basishaftklebemasse sowie für die zuvor beschriebene erwärmbare Haftklebemasse nach Test A ermittelt:

| | |
|---|---|
| Basishaftklebemasse: | 7,4 N/cm |
| Erwärmbare Haftklebemasse: | 6,3 N/cm |

Die Ergebnisse dieses Tests zeigen, dass durch Beimengen eines leitfähigen Füllstoffs zu der Basishaftklebemasse deren haftklebrige Eigenschaften weitgehend erhalten bleiben.

Die Schälkraft (Peelkraft) wurde für die zuvor beschriebene Basisheißschmelzklebemasse sowie für die zuvor beschriebene erwärmbare Heißschmelzklebemasse nach Test B ermittelt:

| | |
|---|---|
| Basisheißschmelzklebemasse: | 4,5 N/cm |
| Erwärmbare Heißschmelzklebemasse: | 3,1 N/cm |

Die Ergebnisse dieses Tests zeigen, dass durch Beimengen eines leitfähigen Füllstoffs zu der Basisheißschmelzklebemasse deren heißschmelzklebrigen Eigenschaften weitgehend erhalten bleiben

Die Erwärmbarkeit und der PTC-Effekt wurden für Beispiel 1 und Vergleichsbeispiel A sowie für Vergleichsbeispiel 1 nach Test C ermittelt. Dabei erreichten die Flächenelemente folgende Temperaturen:

| | |
|---|---|
| Beispiel 1: | 53 °C |
| Vergleichsbeispiel A: | 64 °C |
| Vergleichsbeispiel 1: | 54 °C |

Die Ergebnisse dieses Tests zeigen, dass das erfindungsgemäßen Flächenelemente eine Heizleistung erreicht, die der Heizleistung von derzeit auf dem Markt verfügbaren Autospiegelheizungssystemen aus dem Stand der Technik entspricht.

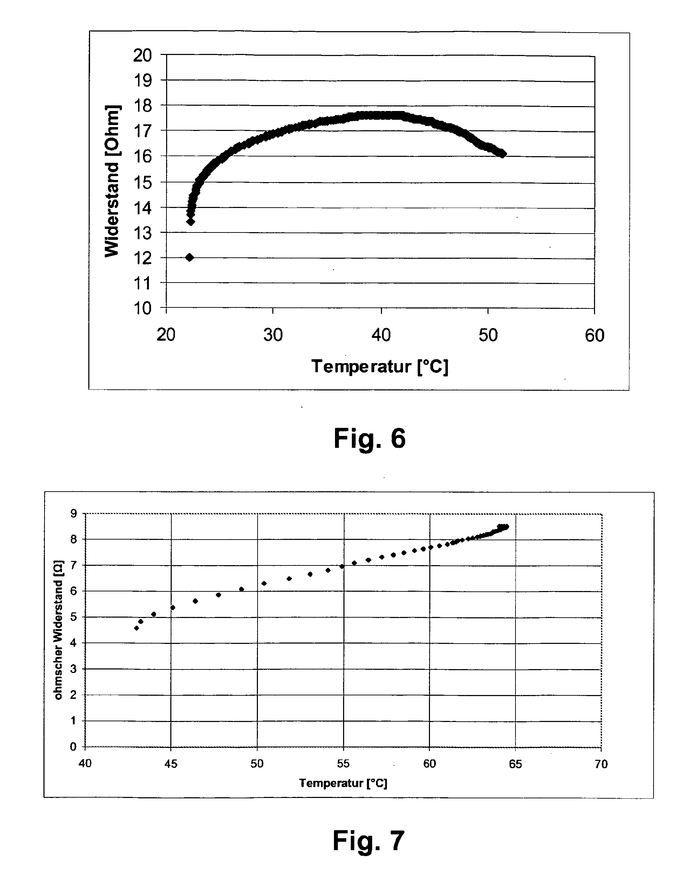

Der aus dem Momentanstrom und der jeweiligen Momentanspannung errechnete Gesamtwiderstand des Flächenelements ist in Fig. 6 und Fig. 8 in Abhängigkeit von der Temperatur wiedergegeben. Die hieraus erhaltene Kurvenform bietet Hinweise auf den PTC-Effekt der Heizschichten. Fig. 6 zeigt die Ergebnisse für Beispiel 1, Fig. 7 die Ergebnisse für Vergleichsbeispiel A und Fig. 8 die Ergebnisse für Vergleichsbeispiel 1. Beim Vergleich der hierbei erhaltenen Messdatenkurven ist erkennbar, dass der PTC-Effekt für die erfindungsgemäßen Flächenelemente zum Teil sogar noch ausgeprägter ist als für das kommerzielle Vergleichsbeispiel.

Die vorstehend beschriebenen exemplarischen Versuche belegen die hervorragende Eignung der erfindungsgemäßen flexiblen Flächenelemente zum Erzielen einer stabilen beheizbaren Verklebung.

**Patentansprüche**

1. Flächenelement mit einer ersten selbstklebenden Seitenfläche und einer zweiten selbstklebenden Seitenfläche, wobei das Flächenelement eine Schichtabfolge aus einer Heizschicht, einer Kontaktierungsschicht und einer Klebemassenschicht aufweist,
   in der die Heizschicht mit einer ersten Seitenfläche der Kontaktierungsschicht in Kontakt steht und mit dieser elektrisch leitend verbunden ist, wobei sich die Heizschicht und die Kontaktierungsschicht unmittelbar berühren, und
   in der die Klebemasseschicht mit einer zweiten Seitenfläche der Kontaktierungsschicht in Kontakt steht und diese unmittelbar berührt, und wobei die Heizschicht aus einer intrinsisch erwärmbaren ersten Selbstklebemasse (10) besteht, die als bei Hindurchleiten eines elektrischen Stroms sich erwärmender Kaltleiter ausgebildet ist, und die Klebemasseschicht aus einer zweiten Selbstklebemasse (30) besteht,
   wobei die erste Selbstklebemasse und die zweite Selbstklebemasse jeweils Haftklebemassen sind, die bei Raumtemperatur eine dauerhafte Verklebung mit einem Substrat eingehen,
   wobei
   die Kontaktierungsschicht ein flächenförmig ausgebreitetes durchbrochenes Kontaktierungselement (20) ist.

2. Flächenelement nach Anspruch 1, **dadurch gekennzeichnet, dass** das durchbrochene Kontaktierungselement (20) Aussparungen aufweist, deren Hauptausdehnung zumindest im Wesentlichen in einer Raumrichtung verläuft.

3. Flächenelement nach Anspruch 1 oder 2, **dadurch gekennzeichnet, dass** das durchbrochene Kontaktierungselement (20) eine verzweigte Kammstruktur oder Fingerstruktur aufweist.

4. Flächenelement nach einem der Ansprüche 1 bis 3, **dadurch gekennzeichnet, dass** alle Teilbereiche des durchbrochenen Kontaktierungselements (20) miteinander durch das durchbrochene Kontaktierungselement (20) elektrisch leitend verbunden sind.

5. Flächenelement nach einem der Ansprüche 1 bis 3, **dadurch gekennzeichnet, dass** das durchbrochene Kontaktierungselement (20) zumindest zwei Teilbereiche aufweist, die nicht miteinander über das durchbrochene Kontaktierungselement (20) elektrisch leitend verbunden sind.

6. Flächenelement nach einem der Ansprüche 1 bis 5, **dadurch gekennzeichnet, dass** die erste Selbstklebemasse (10) mindestens einen elektrisch leitfähigen Füllstoff umfasst.

7. Flächenelement nach Anspruch 6, **dadurch gekennzeichnet, dass** der elektrisch leitfähige Füllstoff ausgewählt ist aus der Gruppe umfassend Graphit, Kohlenstoff-Nanoteilchen und Ruß, insbesondere Leitruß.

8. Flächenelement nach einem der Ansprüche 6 oder 7, **dadurch gekennzeichnet, dass** die erste Selbstklebemasse (10) teilkristalline Polymere aufweist.

9. Flächenelement nach einem der Ansprüche 1 bis 8, **dadurch gekennzeichnet, dass** die erste Selbstklebemasse (10) und/oder die zweite Selbstklebemasse (30) eine Haftklebemasse auf der Basis von Acrylaten und/oder Methacrylaten, Naturkautschuken, Synthesekautschuken und/oder Silikonen ist.

10. Flächenelement nach einem der Ansprüche 1 bis 9, **dadurch gekennzeichnet, dass** die Zusammensetzung der ersten Selbstklebemasse (10) mit der Zusammensetzung der zweiten Selbstklebemasse (30) identisch ist.

11. Flächenelement nach einem der Ansprüche 1 bis 9, **dadurch gekennzeichnet, dass** die Zusammensetzung der ersten Selbstklebemasse (10) von der Zusammensetzung der zweiten Selbstklebemasse (30) verschieden ist.

12. Flächenelement nach einem der Ansprüche 1 bis 11, **dadurch gekennzeichnet, dass** das Flächenelement an der von dem durchbrochenen Kontaktierungselement (20) abgewandten Seitenfläche der Heizschicht eine dritte Selbstklebemasse aufweist.

13. Flächenelement nach einem der Ansprüche 1 bis 12, **dadurch gekennzeichnet, dass** die Heizschicht eine Dicke von weniger als 1 mm aufweist, bevorzugt eine Dicke aus einem Bereich von 10 μm bis 400 μm, besonders bevorzugt aus einem Bereich von 20 μm bis 200 μm.

14. Flächenelement nach einem der Ansprüche 1 bis 13, **dadurch gekennzeichnet, dass** das Flächenelement einen flexiblen permanenten Träger umfasst.

15. Flächenelement nach einem der Ansprüche 1 bis 13, **dadurch gekennzeichnet, dass** das Flächenelement trägerfrei ausgebildet ist.

16. Verklebungsverbund aus einem Verklebungssubstrat und einem Flächenelement nach einem der Ansprüche 1 bis 15, insbesondere mit einer Sichtscheibe oder Spiegelscheibe als Verklebungssubstrat.

17. Verfahren zur Herstellung eines Flächenelements nach einem der Ansprüche 1 bis 15 umfassend die Schritte:

   Ausbilden einer ersten Klebeschichtung,
   Auftragen des durchbrochenen Kontaktierungselements (20) direkt auf die Oberfläche der ersten Klebeschichtung, und
   Auftragen einer zweiten Klebeschichtung auf die Oberfläche des durchbrochenen Kontaktierungselements (20).

18. Verwendung eines Flächenelements nach einem der Ansprüche 1 bis 15 zum Beheizen eines Verklebungsverbunds nach Anspruch 16, insbesondere auf Verklebungssubstraten in der Automobilindustrie.

19. Verwendung eines Flächenelements nach einem der Ansprüche 1 bis 15 zum Verkleben auf der Oberfläche eines menschlichen oder tierischen Körpers, wobei das Flächenelement zumindest einen aktiven Stoff umfasst, der durch Wärme freisetzbar ist oder dessen Freisetzung durch Wärme unterstützt wird.

**Claims**

1. Planar element having a first self-adhesive side face and a second self-adhesive side face, the planar element featuring a layer sequence comprising a heating layer, a contacting layer and an adhesive layer, wherein the heating layer is in contact, and in electrically conducting communication, with a first side face of the contacting layer, where the heating layer and the contacting layer are in direct contact, and wherein the adhesive layer is in contact with a second side face of the contacting layer and is in direct contact therewith, and where

the heating layer is composed of an intrinsically heatable first self-adhesive (10) designed as a posistor which heats up when an electric current is passed through, and

the adhesive layer is composed of a second self-adhesive (30),

where the first self-adhesive and the second self-adhesive are in each case pressure-sensitive adhesives which undergo a permanent adhesive bond with a substrate at room temperature,

where

the contacting layer is a two-dimensionally extended perforate contacting element (20).

2. Planar element according to Claim 1, **characterized in that** the perforate contacting element (20) has reliefs whose principal extent runs at least substantially in one spatial direction.

3. Planar element according to Claim 1 or 2, **characterized in that** the perforate contacting element (20) has a branched comb structure or finger structure.

4. Planar element according to any of Claims 1 to 3, **characterized in that** all of the sub-regions of the perforate contacting element (20) are in electrically conducting connection with one another through the perforate contacting element (20).

5. Planar element according to any of Claims 1 to 3, **characterized in that** the perforate contacting element (20) has at least two sub-regions which are not in electrically conducting connection with one another via the perforate contacting element (20).

6. Planar element according to any of Claims 1 to 5, **characterized in that** the first self-adhesive (10) comprises at least one electrically conductive filler.

7. Planar element according to Claim 6, **characterized in that** the electrically conductive filler is selected from the group encompassing graphite, carbon nanoparticles and carbon black, more particularly conductive carbon black.

8. Planar element according to either of Claims 6 and 7, **characterized in that** the first self-adhesive (10) features partially crystalline polymers.

9. Planar element according to any of Claims 1 to 8, **characterized in that** the first self-adhesive (10) and/or the second self-adhesive (30) is a pressure-sensitive adhesive based on acrylates and/or methacrylates, natural rubbers, synthetic rubbers and/or silicones.

10. Planar element according to any of Claims 1 to 9, **characterized in that** the composition of the first self-adhesive (10) is identical to the composition of the second self-adhesive (30).

11. Planar element according to any of Claims 1 to 9, **characterized in that** the composition of the first self-adhesive (10) is different from the composition of the second self-adhesive (30).

12. Planar element according to any of Claims 1 to 11, **characterized in that** the planar element has a third self-adhesive on the side face of the heating layer that faces away from the perforate contacting element (20).

13. Planar element according to any of Claims 1 to 12, **characterized in that** the heating layer has a thickness of less than 1 mm, preferably a thickness from a range from 10 $\mu$m to 400 $\mu$m, more preferably from a range from 20 $\mu$m to 200 $\mu$m.

14. Planar element according to any of Claims 1 to 13, **characterized in that** the planar element comprises a flexible permanent backing.

15. Planar element according to any of Claims 1 to 13, **characterized in that** the planar element is of backing-free form.

16. Adhesively bonded assembly comprising a bonding substrate and a planar element according to any of Claims 1 to 15, more particularly having a viewing plate or mirror plate as bonding substrate.

17. Method of producing a planar element according to any of Claims 1 to 15, comprising the steps of:

forming a first adhesive stratum,
applying the perforate contacting element (20) directly to the surface of the first adhesive stratum, and
applying a second adhesive stratum to the surface of the perforate contacting element (20).

18. Use of a planar element according to any of Claims 1 to 15 for heating an adhesively bonded assembly according to Claim 16, more particularly on bonding substrates in the automotive industry.

19. Use of a planar element according to any of Claims 1 to 15 for bonding on the surface of a human or animal body, the planar element comprising at least one active substance which can be released by heat or whose release is supported by heat.

**Revendications**

1. Elément plat présentant une première face autoadhésive et une deuxième face autoadhésive, l'élément plat présentant une succession de couches constituée d'une couche chauffante, d'une couche de contact et d'une couche de pâte adhésive,

la couche chauffante étant en contact avec une première face de la couche de contact et étant reliée de manière électriquement conductrice à cette dernière, la couche chauffante et la couche de contact se touchant directement et

la couche de pâte adhésive étant en contact avec une deuxième face de la couche de contact et touchant cette dernière directement,

la couche chauffante étant constituée d'une première pâte autoadhésive (10) intrinsèquement chauffable qui est configurée comme conducteur froid s'échauffant lorsqu'il est traversé par un courant électrique et

la couche de pâte adhésive étant constituée d'une deuxième pâte autoadhésive (30),

la première pâte autoadhésive et la deuxième pâte autoadhésive étant toutes deux des pâtes adhésives qui adhèrent de manière durable à un substrat à température ambiante,

la couche de contact étant un élément de contact (20) perforé s'étendant en surface.

2. Elément de surface selon la revendication 1, **caractérisé en ce que** l'élément de contact (20) perforé présente des découpes dont l'extension principale s'étend au moins essentiellement dans une direction de l'espace.

3. Elément de surface selon les revendications 1 ou 2, **caractérisé en ce que** l'élément de contact (20) perforé présente une structure ramifiée en peigne ou en doigts.

4. Elément de surface selon l'une des revendications 1 à 3, **caractérisé en ce que** toutes les parties de l'élément de contact (20) perforé sont reliées de manière électriquement conductrice les unes aux autres par l'élément de contact (20) perforé.

5. Elément de surface selon l'une des revendications 1 à 3, **caractérisé en ce que** l'élément de contact (20) perforé présente au moins deux parties qui ne sont pas reliées de manière électriquement conductrice l'une à l'autre par l'élément de contact (20) perforé.

6. Elément de surface selon l'une des revendications 1 à 5, **caractérisé en ce que** la première pâte autoadhésive (10) comporte au moins une charge électriquement conductrice.

7. Elément de surface selon la revendication 6, **caractérisé en ce que** la charge électriquement conductrice est sélectionnée dans l'ensemble comportant le graphite, les nanoparticules de carbone et le noir de carbone, en particulier le noir de carbone conducteur.

8. Elément de surface selon l'une des revendications 6 ou 7, **caractérisé en ce que** la première pâte autoadhésive (10) présente des polymères partiellement cristallins.

9. Elément de surface selon l'une des revendications 1 à 8, **caractérisé en ce que** la première pâte autoadhésive (10) et/ou la deuxième pâte autoadhésive (30) sont des pâtes adhésives à base d'acrylates et/ou de méthacrylates, de caoutchoucs naturels, de caoutchoucs de synthèse et/ou de silicones.

10. Elément de surface selon l'une des revendications 1 à 9, **caractérisé en ce que** la composition de la première pâte autoadhésive (10) est identique à la composition de la deuxième pâte autoadhésive (30).

11. Elément de surface selon l'une des revendications 1 à 9, **caractérisé en ce que** la composition de la première pâte autoadhésive (10) est différente de la composition de la deuxième pâte autoadhésive (30).

12. Elément de surface selon l'une des revendications 1 à 11, **caractérisé en ce que** l'élément de surface présente une troisième pâte autoadhésive sur sa face non tournée vers l'élément de contact (20) perforé de la couche chauffante.

13. Elément de surface selon l'une des revendications 1 à 12, **caractérisé en ce que** la couche chauffante présente une épaisseur inférieure à 1 mm, de préférence une épaisseur comprise dans la plage de 10 $\mu$m à 400 $\mu$m et de façon particulièrement préférable dans la plage de 20 $\mu$m à 200 $\mu$m.

14. Elément de surface selon l'une des revendications 1 à 13, **caractérisé en ce que** l'élément plat comporte un support flexible permanent.

15. Elément de surface selon l'une des revendications 1 à 13, **caractérisé en ce que** l'élément de surface est configuré sans support.

16. Ensemble adhésif constitué d'un substrat adhésif et d'un élément de surface selon l'une des revendications 1 à 15, en particulier doté d'une vitre transparente ou d'une vitre réfléchissante comme substrat de collage.

17. Procédé de fabrication d'un élément de surface selon l'une des revendications 1 à 15, comportant les étapes qui consistent à :

    former une première couche adhésive,

appliquer l'élément de contact (20) perforé directement sur la surface de la première couche adhésive et

appliquer une deuxième couche adhésive sur la surface de l'élément de contact (20) perforé.

18. Utilisation d'un élément de surface selon l'une des revendications 1 à 15 pour chauffer un ensemble adhésif selon la revendication 16, en particulier sur des substrats de collage de l'industrie automobile.

19. Utilisation d'un élément de surface selon l'une des revendications 1 à 15 pour être collé sur la surface d'un corps humain ou animal, l'élément de surface comportant au moins une substance active qui peut être libérée par chauffage ou dont la libération est soutenue par la chaleur.

Schnitt X-X

30

20

10

Schnitt Y-Y

Y

Y

X

X

40

21

40

# Fig. 1

Schnitt X-X

Y

30

20

10

Y

+ | - | + | - | + | - | +

Schnitt Y-Y

X

+

-

X

## Fig. 2

16

30

20

10

- | + | - | + | - | +

## Fig. 3

**Fig. 4**

**Fig. 5**

**Fig. 6**

**Fig. 7**

**Fig. 8**

a)

b)

**Fig. 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2948350 A1
- EP 0307205 A1
- EP 0512703 A1
- EP 0852801 A1
- EP 0435923 A1
- DE 10310722 A1
- DE 10310722
- WO 2004081136 A
- DE 102007007617 A
- WO 2008098847 A
- EP 0311142 A1
- US 4775778 A
- EP 04712016 A

**In der Beschreibung aufgeführte Nicht-Patentliteratur**

- **J. MEYER.** *Polymer Engineering and Science,* 1973, vol. 13, 462-468
- **VON DONATAS SATAS.** Handbook of Pressure Sensitive Adhesive Technology. van Nostrand, 1989
- **T.G. FOX.** *Bull. Am. Phys. Soc.,* 1956, vol. 1, 123
- **SKELHORNE.** Electron Beam Processing, in Chemistry and Technology of UV and EB Formulation for Coatings. Inks and Paints, 1991, vol. 1